# EUROPEAN PATENT APPLICATION

(11) **EP 2 695 892 A1**
(43) Date of publication of application: **12.02.2014**
(21) Application number: 12767210.3
(22) Date of filing: 03.04.2012
(51) Int. Cl.: C07K 14/155, A61K 39/21, A61P 31/18, C07K 1/06

(54) **PEPTIDE WHICH CAN INDUCE ANTIBODY CAPABLE OF RECOGNIZING STEREOSTRUCTURE OF HIV**

(30) Priority: 04.04.2011 JP 2011082813
(71) Applicant: National University Corporation Tokyo Medical and Dental University, Bunkyo-ku Tokyo 113-8510 (JP)
(72) Inventor: TAMAMURA, Hirokazu, Tokyo 113-8510 (JP); NARUMI, Tetsuo, Tokyo 113-8510 (JP); NOMURA, Wataru, Tokyo 113-8510 (JP); HASHIMOTO, Chie, Tokyo 113-8510 (JP); KOMANO, Jun A., Tokyo 162-0053 (JP); MIYAUCHI, Kosuke, Tokyo 169-0051 (JP)
(74) Representative: Cabinet Plasseraud
(86) International application number: PCT/JP2012/002312
(87) International publication number: WO 2012/137479

(57) **Abstract**

An object of the present invention is to provide a peptide capable of inducing a superior or new neutralizing antibody against HIV, so that HIV infectious disease can be prevented and treated or a greater variety of preventive or therapeutic options can be offered. This object is achieved by using a peptide inducing an HIV's three-dimensional structure-recognizing antibody that recognizes a trimer region of C34, wherein three molecules of a derivative of a helical region C34 peptide at C-terminal region of transmembrane protein gp41 of an HIV particle are ligated via a C3-symmetric template compound having three equivalent linker structures.

## Description

### TECHNICAL FIELD

The present invention relates to peptides capable of inducing antibodies that recognize the three-dimensional structure of HIV (human immunodeficiency virus), methods for synthesizing the same, and preventive and/or therapeutic agents for HIV infectious disease which comprise as an active ingredient said peptides or HIV's three-dimensional structure-recognizing antibodies induced by said peptides. Particularly, the present invention relates to peptides capable of inducing antibodies that recognize the three-dimensional structure of HIV, wherein the hexamerization of N36 and a C-terminal helical region C34 in transmembrane protein gp41 of an HIV particle, i.e., the mechanism by which HIV invades a target cell, is inhibited by said peptides in an HIV vaccine which act on a trimer region of the helical region C34 to prevent HIV invasion into the target cell, methods for synthesizing the same, and preventive and/or therapeutic agents for HIV infectious disease which comprise as an active ingredient said peptides or HIV's three-dimensional structure-recognizing antibodies induced by said peptides.

### BACKGROUND ART

Acquired immunodeficiency syndrome (AIDS) is a disease caused by human immunodeficiency virus (HIV) which was isolated and discovered by Montagnier L. et al. in 1983 (see Non-Patent Document 1). The origin of HIV has been considered to be the ability to infect a human, acquired due to mutation by simian immunodeficiency virus (SIV) whose natural host is a primate. HIV infects hosts not by airborne infection but through three main pathways: sexual transmission, blood infection, and mother-to-child transmission. The greatest cause of the sexual transmission is deemed to be contact with sexual discharges. The blood infection is caused by transfusion, wounds, needle-sharing, and the like. Therefore, infection prevention from an ethical standpoint is required. Moreover, it is alleged that the mother-to-child transmission is mainly caused by contact with body fluids in the birth canal during parturition, maternal breastfeeding, and virus movement through the placenta during pregnancy.

HIV, a species of retrovirus, is an adventitious virus that particularly targets and infects human CD4-positive T cells (see Non-Patent Document 1). HIV that has infected the human CD4-positive T cells is activated after a relatively long incubation period to destroy the T cells. Since T cells play an important role in the immune system, the immune capacity of the body is significantly reduced if the T cells are destroyed. As a result, the body can no longer exhibit sufficient resistance even to pathogens that should be eliminated easily if it is in a normal state, and as the result, the body falls into a chronic immunodeficiency, i.e., a state called "onset of AIDS".

The number of HIV-infected people has already reached 33 million throughout the world, though the speed at which the number of HIV infections increases is getting slower. In 2007, the number of new HIV infections was 2.4 million, and the number of deaths related to HIV was 2 million (see Non-Patent Document 2). Particularly, in Asia which has the largest population in the world, HIV infection has rapidly been spreading. Japan is no exception and the number of HIV-infected people is increasing steadily, which is an unusual case among developed countries. Specifically, in 2007, the number of AIDS patients was 418, and the number of HIV-infected people was 1082, exceeding 1000 for the first time. Under the present circumstances, such increase remains to be controlled in Japan (see Non-Patent Document 3). However, HIV has been studied actively since its discovery. During the 25 years that passed to the present, a method for diagnosing the infectious disease has been established, and overwhelming progress is also seen in therapy, compared with other infectious diseases (see Non-Patent Documents 4 and 5). By virtue of energetic R&D efforts on therapeutic drugs, AIDS is no longer a disease directly leading to death. However, radical therapy has not been established yet, and new problems have also emerged. Therefore, there is a demand for novel therapeutic drugs.

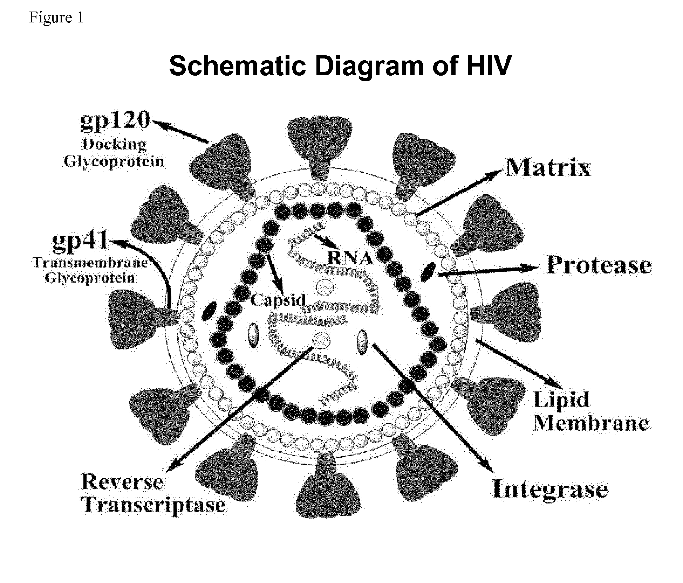

HIV has envelope proteins called gp120 and gp41 on a viral membrane derived from a host cell and these proteins are necessary for binding to a target cell. A matrix protein exists as a scaffold protein on the inner surface of the viral membrane and helps maintain the HIV's structure. Moreover, the nucleoid of HIV has a regular dodecahedron structure surrounded by capsid proteins and internally contains the RNA genome, integrase (IN), protease (PR), and reverse transcriptase (RT) (Figure 1). The RNA genome of HIV is approximately 9000 bp long, and the gene cluster is flanked by structures called long terminal repeats (LTRs). A dozen kinds of viral proteins encoded by these genes control complicated replication (see Non-Patent Documents 4 and 5).

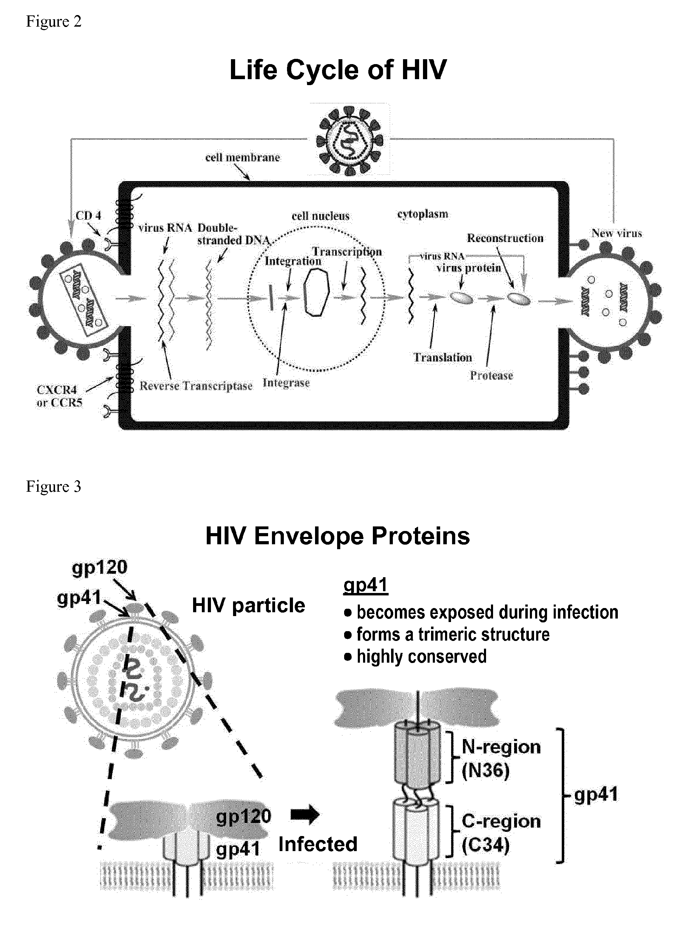

A series of multiplication cycles from HIV invasion into a target cell to budding is called life cycle. This life cycle is divided into the following stages (1) to (6): (1) adsorption of the virus onto the cell membrane and membrane fusion; (2) reverse transcription of the RNA genome; (3) viral DNA integration into the host chromosome and replication; (4) processing of the constituent proteins of the virus; (5) construction of virions; and (6) budding. HIV multiplies through these stages (see Figure 2) (see Non-Patent Documents 4 to 7).

Azidothymidine (AZT), the first anti-HIV drug developed in 1985, is a competitive inhibitor of reverse transcriptase. This drug inhibits HIV multiplication by inhibiting the above-described life cycle. Since the development of this AZT, the life cycle of HIV has been revealed in more detail, and the development of anti-HIV drugs has drastically progressed (see Non-Patent Documents 8 and 9). As a result, no less than 15 kinds of anti-HIV drugs are currently under clinical application and have enabled highly active anti-retroviral therapy (HAART) in which anti-HIV drugs having different mechanisms of action are used in combination. HAART is currently a mainstream treatment of HIV-infected people and AIDS patients and certain effects have been attained. However, the long-term administration of drugs is required for continuously reducing virus loads in the body as much as possible. As a result, there also arise problems such as emergence of drug-resistant strains, a serious adverse reaction, and the need for expensive medical fees for treatment. The emergence of drug-resistant viruses is a particularly serious problem. Causes of the emergence of drug-resistant viruses include long-term medication as well as the mutability of HIV (which is highly prone to mutation since it has no DNA repair mechanism such as DNA polymerase).

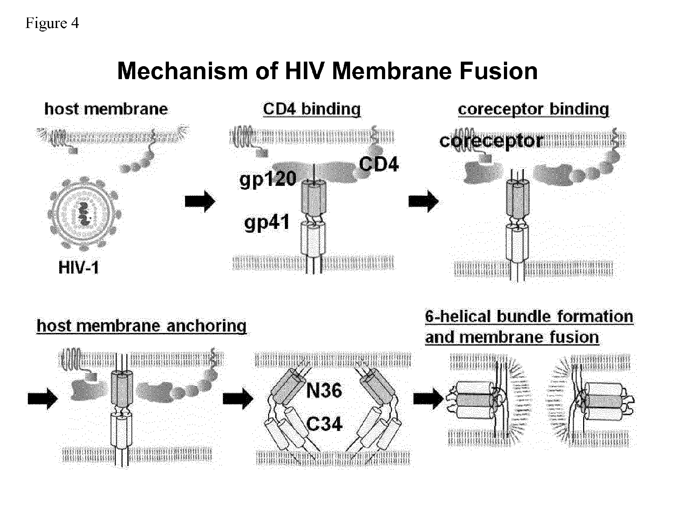

Under the circumstances, it is desired to develop preventive agents and therapeutic agents for HIV infectious disease that require less medical fees for treatment and which yet are effective against resistant HIV virus. Candidates for preventive and therapeutic agents for HIV infectious disease that are based on (1) of the above-mentioned HIV's life cycle, i.e., "adsorption of the virus onto the cell membrane and membrane fusion" and which are currently under development are those which are targeted to the HIV's envelope proteins gp120 and gp41. These envelope proteins are involved in cell fusion that occurs when HIV invades a host cell. Before infection, the greater part of gp41 is coated with gp120 (see the left panel of Figure 3) but becomes exposed in the process of infection (see the right panel of Figure 3.) The N-terminal region of gp41 is called N36 and the C-terminal region C34, each of N36 and C34 forming a trimeric structure. The mechanism of membrane fusion between HIV and host cell is shown in Figure 4. When HIV invades to a host cell, in the first step, gp120 binds to CD4 in the host cell and then binds to a coreceptor. Thereafter, gp41 is spiked to the host cell membrane, whereupon a three-dimensional structural change occurs. Then, binding takes place in such a way that three C34 chains surround three N36 chains from the outside to form a 6-helical bundle consisting of six associated peptide chains, whereupon the HIV membrane and the host cell membrane are brought sufficiently close to each other to establish membrane fusion (see Figure 4).

Based on this mechanism of HIV invasion, several antibodies have been induced that target gp120 or gp41 and have an anti-HIV activity. Currently, 2F5 antibody (Non-Patent Documents 10-13) and 4E10 antibody (Non-Patent Documents 12-14) as antibodies against gp41, as well as 2G12 antibody (Non-Patent Document 15) and b12 antibody (Non-Patent Document 16) as antibodies against gp120 are known as human monoclonal antibodies that exhibit a relatively strong anti-HIV activity (HIV neutralizing activity). Furthermore, Patent Document 1 describes the use of antibodies against gp120 for preventing HIV infection or for inactivating a stage essential for the life cycle of HIV. However, these antibodies, albeit with an anti-HIV activity against a certain HIV strain, did not exhibit a sufficient anti-HIV activity against other strains, probably because the HIV genome is significantly mutable. Thus, the antibodies still remain to be applied clinically.

The present inventors already successfully synthesized a peptide antigen capable of inducing a neutralizing antibody that would specifically recognize the trimeric structure of N36 peptide interacting with the C34 peptide of gp41 (Patent Document 2.) This peptide antigen is an antigen molecule that is a C3- symmetric template compound with three equivalent linker structures to each of which a derivative of N36 peptide is individually ligated. A neutralizing antibody induced by this N36 peptide trimeric antigen had higher specificity for the N36 peptide trimer than a neutralizing antibody induced by an N36 peptide monomer and its anti-HIV activity was three times higher. The antibody induced by the N36 peptide trimeric antigen in the above-mentioned Patent Document 2 is considered to inhibit HIV membrane fusion by specifically binding to the trimer of N36 peptide so as to inhibit the 6-helical bundle formation due to N36 peptide and C34 peptide. In addition, the C34 peptide derivative monomer fuzeon (T-20/DP178) is a known anti-HIV active peptide that has been put to clinical trial (Non-Patent Document 17.)

### CITATION LIST

### PATENT DOCUMENTS

Patent Document 1: Japanese Unexamined Patent Application Publication No. 2009-080118
Patent Document 2: pamphlet of WO/2010/134305

### NON-PATENT DOCUMENTS

Non-Patent Document 1: Sinoussi, B. F., Montagnier, L. Isolation of a T-lymphotropic retrovirus from a patient at risk for acquired immune deficiency syndrome (AIDS). Science 220, 868-871 (1983)
Non-Patent Document 2: WHO, World Health Statistics 2008
Non-Patent Document 3: The report of the AIDS Surveillance Committee, the Ministry of Health, Labour and Welfare Japan, "Report on Trend of AIDS Incidence" issued in 2008
Non-Patent Document 4: "Frontiers of Human Retrovirus Research" ed. by Naoki Yamamoto, issued on Feb. 22, 2002, Springer-Verlag Tokyo, Inc.
Non-Patent Document 5: Koyanagi, Y. Outline of the HIV replication and its cellular: the track of an invader in cell. Virus 55, 251-258 (2005)
Non-Patent Document 6: Carter, C. A., Ehrlich, L. S. Cell biology of HIV-1 infection of macrophages. Annu. Rev. Microbiol. 62, 425-443 (2008)
Non-Patent Document 7: Eckert, D. M., Kim, P. S. Mechanisms of viral membrane fusion and its inhibition. Annu. Rev. Biochem. 70, 777-810 (2001)
Non-Patent Document 8: Baba, M. Recent progress of anti-HIV-1 research. Virus 54, 59-66 (2004)
Non-Patent Document 9: Kwong, P. D., Wyatt, R., Robinson, J. Structure of an HIV gp120 envelope glycoprotein in complex with the CD4 receptor and a neutralizing human antibody. Nature 393, 648-659 (1998)
Non-Patent Document 10: Conley, A. J., Kessler, J. A. II, Boots, L. J., Tung, J. S., Arnold, B. A., Keller, P. M., Shaw, A. R., and Emini, R. A. Neutralization of divergent human immunodeficiency virus type 1 variants and primary isolates by IAM-41-2F5, an anti-gp41 human monoclonal antibody Proc. Natl. Acad. Sci. U.S.A. 91, 3348-3352 (1994)
Non-Patent Document 11: Ofek, G., Tang, M., Sambor, A., Katinger, H., Mascola, J. R., Wyatt, R., and Kwong, P. D. Structure and mechanistic analysis of the anti-human immunodeficiency virus type 1 antibody 2F5 in complex with its gp41 epitope. J. Virol. 78, 10724-10737 (2004)
Non-Patent Document 12: Alam, S. M., McAdams, M., Boren, D., Rak, M., Scearce, R. M., Gao, F., Camacho, Z. T., Gewirth, D., Kelsoe, G., Chen, P., and Haynes, B. F. The role of antibody polyspecificity and lipid reactivity in binding of broadly neutralizing anti-HIV-1 envelope human monoclonal antibodies 2F5 and 4E10 to glycoprotein 41 membrane proximal envelope epitopes. J. Immunol. 178, 4424-4435 (2007)
Non-Patent Document 13: Nelson, J. D., Brunel, F. M., Jensen, R., Crooks, E. T., Cardoso, R. M. F., Wang, M., Hessell, A., Wilson, I. A., Binley, J. M., Dawson, P. E., Burton, D. R., and Zwick, M. B. An affinity-enhanced neutralizing antibody against the membrane-proximal external region of human immunodeficiency virus type 1 gp41 recognizes an epitope between those of 2F5 and 4E10. J. Virol. 81, 4033-4043 (2007)
Non-Patent Document 14: Cardoso, R. M. F., Zwick, M. B., Stanfield, R. L., Kunert, R., Binley, J. M., Katinger, H., Burton, D. R., and Wilson, I. A. Broadly neutralizing anti-HIV antibody 4E10 recognizes a helical conformation of a highly conserved fusion-associated motif in gp41. Immunity 22, 163-173 (2005)
Non-Patent Document 15: Trkola, A., Purtscher, M., Muster, T., Ballaun, C., Buchacher, A., Sullivan, N., Srinivasan, K., Sodroski, J., Moore, J. P., and Katinger, H. Human monoclonal antibody 2G12 defines a distinctive neutralization epitope on the gp120 glycoprotein of human immunodeficiency virus type 1. J. Virol. 70, 1100-1108 (1996)
Non-Patent Document 16: Pantophlet, R., Saphire, E. O., Poignard, P., Parren, P. W. H. I., Wilson, I.A., and Burton, D. R. Fine mapping of the interaction of neutralizing and nonneutralizing monoclonal antibodies with the CD4 binding site of human immunodeficiency virus type 1 gp120. J. Virol. 77, 642-658 (2003)
Non-Patent Document 17: Akira Otaka, Miki Nakamura, Daisuke Nameki, Eiichi Kodama, Susumu Uchiyama, Syota Nakamura, Hiroaki Nakano, Hirokazu Tamamura, Yuji Kobayashi, Masao Matsuoka, and Nobutaka Fujii Remodeling of gp41-C34 Peptide Leads to Highly Effective Inhibitors of the Fusion of HIV-1 with Target Cells. Angew. Chem. Int. Ed., 41(16), 2937-2940 (2002)

### SUMMARY OF INVENTION

### TECHNICAL PROBLEM

A problem of the development of HIV vaccines is that the approaches effective for traditional infectious diseases, such as attenuated vaccines and live vaccines, are regarded as dangerous due to the mutability of HIV and, therefore, can no longer be used. Moreover, antibody induction is usually performed by synthesizing a partial sequence of the protein of HIV for the purpose of inducing antibodies specific for that partial sequence. The antibodies induced using such a partial sequence are capable of specifically binding to the amino acid sequence but their problem is that the specificity or binding activity for the three-dimensional structure of a neutralization target is generally low. There is desired an HIV antibody-inducing peptide that can be used without problems against such highly mutatable HIV and which are effective in the development of preventive or therapeutic agents for HIV infectious disease, such as an antibody or a vaccine having superior specificity and binding activity even for the three-dimensional structure of a neutralization target, i.e. the mechanism by which HIV invades a target cell. As such peptide, the present inventors previously developed a peptide antigen capable of inducing a neutralizing antibody that would specifically recognize the trimeric structure of N36 peptide in gp41 of HIV (see Patent Document 2).

However, a peptide capable of inducing a superior or new neutralizing antibody effective against HIV is desired for preventing or treating HIV infectious disease or for providing a greater variety of preventive or therapeutic options.

### SOLUTION TO PROBLEM

The present inventor has conducted diligent studies to attain the above-stated object and consequently had the idea that an antibody superior in anti-HIV activity or neutralizing activity against HIV infection could be induced by chemically synthesizing a trimer of an important portion called C34 in the helical region of gp41 and using it as a peptide capable of inducing an antibody that recognizes an HIV's three-dimensional structure. Based on this finding, the present invention has been completed.

Specifically, the present invention relates to the following: [1] a method for synthesizing a peptide inducing an HIV's three-dimensional structure-recognizing antibody that recognizes a trimer region of C34, the method comprising: the steps of synthesizing a derivative of a helical region C34 peptide at C-terminal region of transmembrane protein gp41 of an HIV particle, and synthesizing a trimer of the C34 peptide derivative by ligating the C34 peptide derivative to a C3-symmetric template compound having three equivalent linker structures; [2] the method for synthesizing a peptide inducing an HIV's three-dimensional structure-recognizing antibody as recited in [1] above, wherein the C34 peptide derivative in the step of synthesizing a derivative of a helical region C34 peptide at C-terminal region of transmembrane protein gp41 of an HIV particle is such that a ligation site necessary for ligation with the template compound is introduced at the C-terminal region of a C34 peptide that is the native sequence of C34 whereas spacer amino acid residue(s) for preventing reduced reactivity due to steric hindrance is introduced between the sequence of said C34 peptide and said ligation site; [3] the method for synthesizing a peptide inducing an HIV's three-dimensional structure-recognizing antibody as recited in [1] or [2] above, wherein a compound represented by the following general formula (1) or a salt thereof is used as the C3-symmetric template compound having three equivalent linker structures 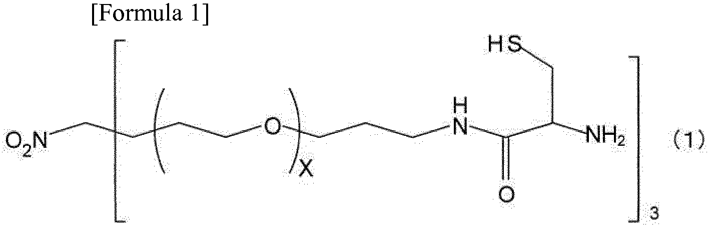 (where X is a positive integer);
[4] the method for synthesizing a peptide inducing an HIV's three-dimensional structure-recognizing antibody as recited in [3] above, wherein a compound represented by the following general formula (2) or a salt thereof is used as the C3-symmetric template compound having three equivalent linker structures 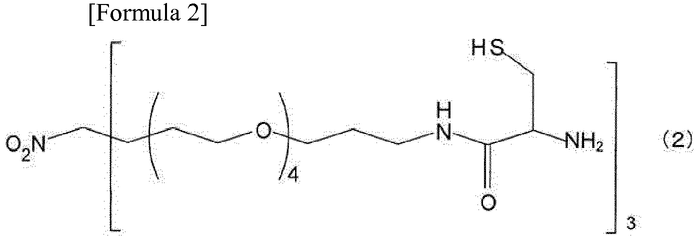
[5] the method for synthesizing a peptide inducing an HIV's three-dimensional structure-recognizing antibody as recited in [1] above, wherein the C34 peptide derivative in the step of synthesizing a derivative of a helical region C34 peptide at C-terminal region of transmembrane protein gp41 of an HIV particle is such that hydrophilic amino acid residue(s) is imparted at the C terminus of the C34 peptide, a ligation site is further introduced at C terminus thereof whereas spacer amino acid residue(s) for preventing reduced reactivity due to steric hindrance is introduced between said hydrophilic amino acid residue(s) and said ligation site; [6] the method for synthesizing a peptide inducing an HIV's three-dimensional structure-recognizing antibody as recited in [5] above, wherein the C34 peptide derivative in the step of synthesizing a derivative of a helical region C34 peptide at C-terminal region of transmembrane protein gp41 of an HIV particle is a compound represented by the following general formula (3) 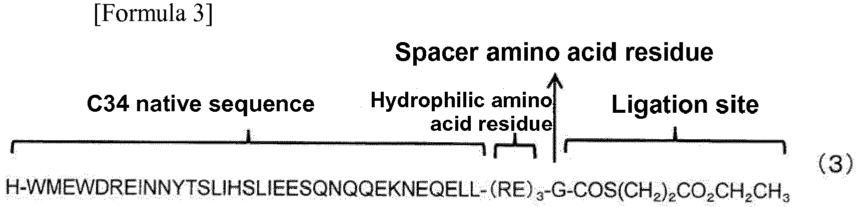
[7] the method for synthesizing a peptide inducing an HIV's three-dimensional structure-recognizing antibody as recited in any one of [1] to [6] above, wherein the step of synthesizing a trimer of the C34 peptide derivative by ligating the aforementioned C34 peptide derivative to a C3-symmetric template compound having three equivalent linker structures is such that the C3-symmetric template compound having three equivalent linker structures and the C34 peptide derivative are stirred in a buffer, whereby the C3-symmetric template compound having three equivalent linker structures and the aforementioned C34 peptide derivative are ligated to synthesize a trimer of the C34 peptide derivative.

The present invention also relates to [8] a peptide inducing an HIV's three-dimensional structure-recognizing antibody that recognizes a trimer region of a helical region C34 at C-terminal region of transmembrane protein gp41 of an HIV particle, the peptide being synthesized by the method of synthesis as recited in any one of [1] to [7] above; [9] a peptide inducing an HIV's three-dimensional structure-recognizing antibody that recognizes a trimer region of C34, wherein three molecules of a derivative of a helical region C34 peptide at C-terminal region of transmembrane protein gp41 of an HIV particle are ligated via a C3-symmetric template compound having three equivalent linker structures; and [10] the peptide inducing an HIV's three-dimensional structure-recognizing antibody as recited in [8] or [9] above, which is a compound represented by the following general formula (4) or a salt thereof 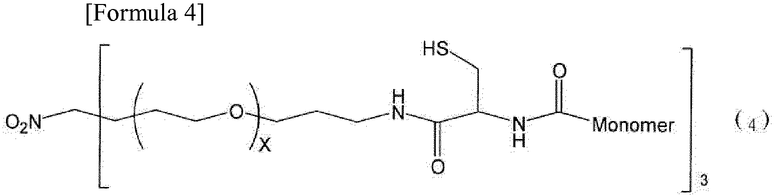 (where X is a positive integer, and Monomer signifies a compound represented by the following general formula (5))

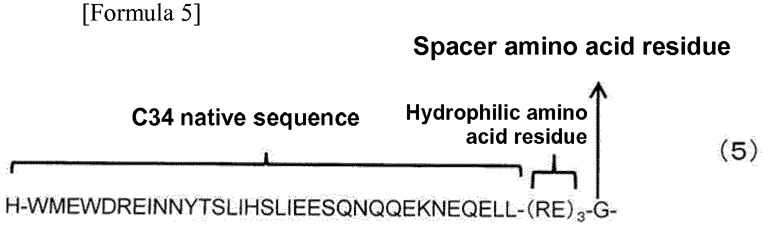

The present invention further relates to [11] a method for inducing an HIV's three-dimensional structure-recognizing antibody that recognizes a trimer region of C34, wherein a host animal is sensitized with a peptide inducing the HIV's three-dimensional structure-recognizing antibody that recognizes a trimer region of C34 as recited in any one of [8] to [10] above, whereby an antibody against said peptide is induced.

The present invention also relates to [12] a preventive and/or therapeutic agent for HIV infectious disease comprising as an active ingredient an HIV's three-dimensional structure-recognizing antibody that recognizes a trimer region of C34 as induced by the peptide recited in any one of [8] to [10] above or the peptide as recited in any one of [8] to [10] above; [13] the preventive and/or therapeutic agent for HIV infectious disease as recited in [12] above, wherein prevention and/or treatment of HIV infectious disease is due to the anti-HIV activity of the peptide as recited in any one of [8] to [10] above; [14] the preventive and/or therapeutic agent for HIV infectious disease as recited in [12] above, wherein prevention and/or treatment of HIV infectious disease is due to the action of an HIV's three-dimensional structure-recognizing antibody on a trimer region of a helical region C34 at C-terminal region of transmembrane protein gp41 of an HIV particle, whereby C34/N36 hexamerization of gp41 is inhibited to block the invasion of HIV into a target cell; and [15] the preventive and/or therapeutic agent for HIV infectious disease as recited in [12] above, which is a preventive and/or therapeutic vaccine for HIV infectious disease comprising as an active ingredient the peptide recited in any one of [8] to [10] above.

### ADVANTAGEOUS EFFECTS OF INVENTION

The present invention can provide an HIV antibody-inducing peptide that is effective in the development of an antibody or vaccine having superior specificity and binding activity for the three-dimensional structure of a neutralization target, i.e., the mechanism by which HIV invades a target cell. The HIV antibody-inducing peptide antigen provided by the present invention enables production of an HIV's three-dimensional structure-recognizing antibody or a vaccine and can provide a preventive and/or therapeutic agent for HIV infectious disease comprising the peptide, the vaccine, or the HIV's three-dimensional structure-recognizing antibody as an active ingredient. A problem of the development of HIV vaccines is that the approaches effective for traditional infectious diseases, such as attenuated vaccines and live vaccines, are regarded as dangerous due to the mutability of HIV and, therefore, can no longer be used. However, the HIV antibody-inducing peptide provided by the present invention is an HIV antibody-inducing peptide that is effective in the development of an antibody or a vaccine having specificity and binding activity not only for the targeted amino acid sequence of highly mutatable HIV but for the three-dimensional structure of a neutralization target, i.e., the mechanism by which HIV invades a target cell. Thus, the above-described problem with the conventional development of HIV vaccines can be solved, and an HIV antibody or HIV vaccine that is safe and effective against HIV infectious disease can be provided. As a result, a preventive and/or therapeutic agent for HIV infectious disease that is safe and effective against HIV infectious disease can be provided.

Referring to the N36 peptide derivative disclosed in Patent Document 2, the anti-HIV activity of its trimer is three times higher than that of its monomer. In contrast, the trimer of the C34 peptide derivative according to the present invention has an anti-HIV activity that is improved as many as 20 to 100 times over its monomer. Since such a high anti-HIV activity is displayed by the HIV antibody inducing peptide of the present invention on its own, a preventive and/or therapeutic agent for HIV infectious disease that comprises the peptide as an active ingredient is capable of exhibiting its efficacy even in a state that is yet to pass through antibody induction, for example, before an antibody is induced.

### BRIEF DESCRIPTION OF DRAWINGS

Figure 1 is a diagram showing a schematic view of HIV.
Figure 2 is a diagram showing the life cycle of HIV.
Figure 3 is a diagram showing the envelope proteins of HIV.
Figure 4 is a diagram showing the mechanism of HIV membrane fusion.
Figure 5 is a diagram showing an outline of native chemical ligation reaction.
Figure 6 is a diagram showing the mechanism of formation of a C34 peptide derivative trimer (C34 Trimer).
Figure 7 is a diagram showing the design of a C34 peptide derivative trimer (C34 Trimer).
Figure 8 is a diagram related to the synthesis of C34 peptide derivative 1 (C34 derivative 1).
Figure 9 is a diagram related to the synthesis of C34 peptide derivative 2 (C34 derivative 2).
Figure 10 is a diagram related to the synthesis of a template compound.
Figure 11 is a diagram showing a synthesis scheme for the template compound.
Figure 12 is a diagram showing the course of reaction involved in the formation of C34 peptide derivative trimer (C34 Trimer).
Figure 13 is a diagram related to the synthesis of C34 peptide derivative trimer (C34 Trimer).

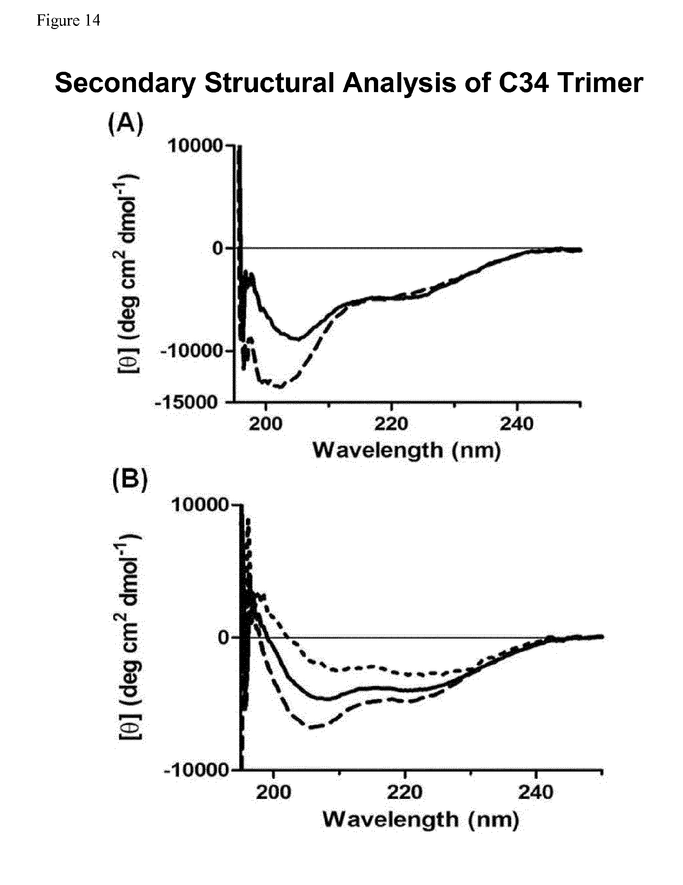

Figure 14 shows graphically the results of a secondary structural analysis on C34 peptide derivative trimer (C34 Trimer); the solid line in panel (A) indicates a CD spectrum for C34 peptide derivative trimer and the dashed line in panel (A) indicates a CD spectrum for C34 peptide derivative monomer; the dashed line in panel (B) indicates a CD spectrum for a mixture of C34 peptide derivative monomer and N36 peptide derivative monomer, the solid line in panel (B) indicates a CD spectrum for a mixture of C34 peptide derivative trimer and N36 peptide derivative monomer, and the dotted line in panel (B) indicates a CD spectrum for N36 peptide derivative monomer.

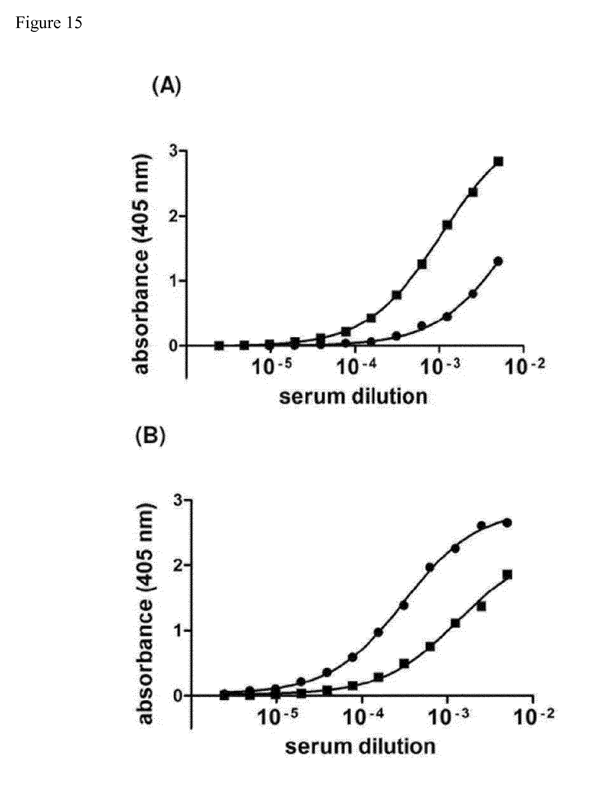

Figure 15 is a diagram showing the antibody titers of sera obtained by immunization with C34 peptide derivative trimer (C34 Trimer) and C34 peptide derivative monomer; panel (A) show the results of a case where microplates were coated with C34 peptide derivative monomer; panel (B) shows the results of a case where microplates were coated with C34 peptide derivative trimer; the solid squares in panels (A) and (B) indicate the results of using sera obtained by induction with C34 peptide derivative monomer whereas the dots indicate the results of using sera obtained by induction with C34 peptide derivative trimer.
Figure 16 is a graph showing the neutralizing activities against HIV infection of anti-sera induced by C34 peptide derivative trimer; "unif." refers to the result from uninfected cells; "cr1" to "cr3" refer to the results from the use of serum one week before immunization (control); "m1" to "m3" refer to the results from sera induced by C34 peptide derivative monomer; "t1" to "t3" refer to the results from sera induced by C34 peptide derivative trimer.
Figure 17 is a graph showing the anti-HIV activities against HIV infection of C34 peptide derivative trimer; "C34" refers to the result of using C34 native peptide monomer; "C34 REG" refers to the result of using C34 peptide derivative monomer; "triC34e" refers to the result of using C34 peptide derivative trimer; the horizontal axis plots the concentration (µM) of each peptide and the vertical axis plots the p24 level (ng/mL) in each case.

### DESCRIPTION OF EMBODIMENTS

### 1. Method for synthesizing peptides inducing an HIV's three-dimensional structure-recognizing antibody

The method of the present invention for synthesizing peptides inducing an HIV's three-dimensional structure-recognizing antibody (hereinafter sometimes referred to simply as the "peptide synthesis method of the present invention") is a method for synthesizing a peptide inducing an HIV's three-dimensional structure-recognizing antibody that recognizes a trimer region of C34, the method comprising the steps of synthesizing a derivative of a helical region C34 peptide at C-terminal region of transmembrane protein gp41 of an HIV particle (hereinafter sometimes referred to simply as a "C34 peptide derivative") and synthesizing a trimer of the C34 peptide derivative by ligating the aforementioned C34 peptide derivative to a C3-symmetric template compound having three equivalent linker structures (hereinafter sometimes referred to simply as a "template compound according to the present invention").

The C34 peptide derivative according to the present invention means peptides that consist of an amino acid sequence derived from the amino acid sequence of the C34 peptide (SEQ ID NO: 1) by the substitution, deletion, or insertion of one or at least two (preferably 2 to 15, more preferably 2 to 10, even more preferably 2 to 5) amino acids and which can be used in the synthesis of a peptide inducing an HIV's three-dimensional structure-recognizing antibody. Advantageous examples of such C34 peptide derivative include peptides obtained by placing a ligation site (advantageously a thioester group) that can be ligated to the template compound according to the present invention at the C-terminal region of the C34 peptide. More advantageous examples include peptides obtained by introducing 1 to 20 amino acid residues, advantageously 1 to 10 amino acid residues, more advantageously 1 to 5 amino acid residues, and even more advantageously one amino acid residue (the residue is advantageously Gly) between the C terminus of the sequence of C34 peptide and the ligation site as a spacer for preventing reduced reactivity due to steric hindrance, and peptides obtained by introducing 1 to 20, advantageously 2 to 18, more advantageously 3 to 12, and even more advantageously 6 hydrophilic amino acid residues (the residue is advantageously Arg or Glu) between the C terminus of the sequence of C34 peptide and the ligation site for providing enhanced water solubility; among others, peptides obtained by introducing both the above-mentioned spacer amino acid residues and hydrophilic amino acid residues are particularly advantageous; more advantageous is a peptide in which the sequence of C34 peptide, hydrophilic amino acid residue(s), spacer amino acid residue(s), and a ligation site are arranged in this order from the N-terminus, and a compound represented by the following general formula (3) is even more advantageous:

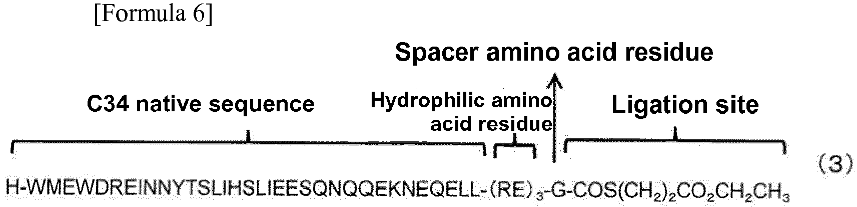

To determine whether a certain C34 peptide derivative is a peptide that can be used in the synthesis of the peptide capable of inducing an HIV's three-dimensional structure-recognizing antibody, it may be examined for the ability to induce an HIV's three-dimensional structure-recognizing antibody that recognizes a trimer region of C34.

In the step of synthesizing a C34 peptide derivative in the peptide synthesis method of the present invention, the C34 peptide derivative can be synthesized using Fmoc solid-phase peptide synthesis (SPPS) (see Hirokazu Tamamura et al., Bioorganic & Medicinal Chemistry 6 (1998), 1033-1041; and "Solid phase peptide synthesis-a practical approach" by E Atherton & R C Sheppard IPI PRESS). Specifically, the peptides of interest can be synthesized by the solid-phase synthesis method by repeating the procedures of selective deprotection of 9-fluorenylmethoxycarbonyl (Fmoc), a protective group for the amino group of the principal chain, and condensation reaction, finally followed by cleavage from resin and deprotection.

In the present invention, the template compound according to the present invention is used in the step of synthesizing a trimer of the C34 peptide derivative. Advantageous examples of the template compound according to the present invention include a compound represented by the following general formula (1) (where X is a positive integer) or a salt thereof:

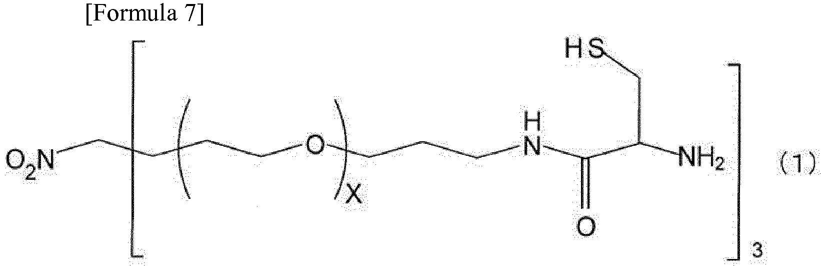

The symbol X is preferably an integer within the range of 1 to 10 from the viewpoint of obtaining a trimer of the C34 peptide derivative that is more analogous to the trimeric structure of C34 in the native gp41 protein; X is more preferably an integer within the range of 2 to 8, even more preferably an integer within the range of 2 to 6, still more preferably an integer in the range of 3 to 5, and particularly preferably 4. By using such a template compound, the C34 peptide derivative can be trimerized conveniently into a structure analogous to the native structure. The Cys moiety at the end of the template compound according to the present invention can be used in native chemical ligation with the C34 peptide derivative.

The template compound according to the present invention may be in the form of a salt such as an acid-addition salt or a base-addition salt. Examples of the acid-addition salt include: mineral acid salts such as hydrochloride, phosphate, nitrate, sulfate, acetate, propionate, butyrate, valerate, citrate, fumarate, maleate, and malate; and organic acid salts such as oxalate or tartrate. Examples of the base-addition salt include: metal salts such as sodium salt, potassium salt, magnesium salt, or calcium salt; ammonium salt; or organic amine salts such as triethylamine salt or ethanolamine salt. Furthermore, the template compound according to the present invention may have any one or at least two substituents as long as it can be used in the present invention. When the template compound according to the present invention has two or more substituents, they may be the same or different. The position at which each substituent is located is not limited as long as it can be used in the present invention. The substituents can be located at any site where substitution is possible. The types of the substituents are not particularly limited. The template compound according to the present invention can be prepared by, for example, the method described in Example 1(3) to be given later.

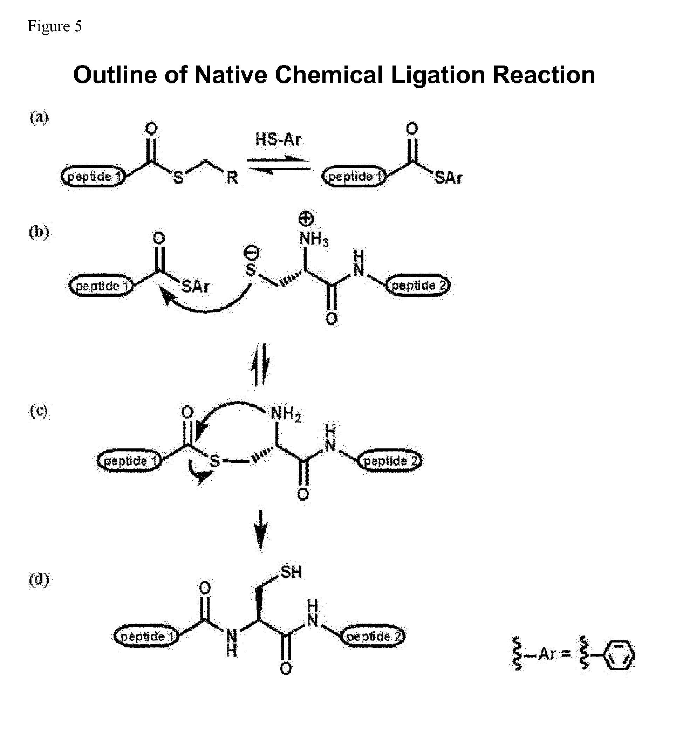

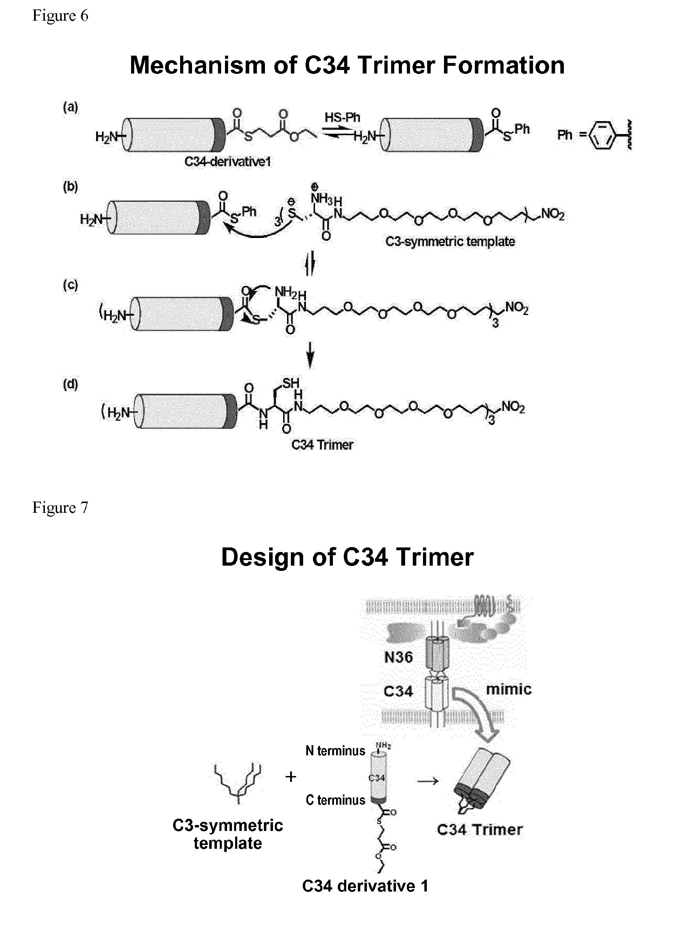

In the step of synthesizing a trimer of the C34 peptide derivative by ligating the C34 peptide derivative of the present invention to the template compound according to the present invention, native chemical ligation reaction can be used to form the trimer of the C34 peptide derivative (Dawson, P. E., Muir, T. W., Clark-Lewis, I., and Kent, S. B. H. (1994) Synthesis of proteins by native chemical ligation. Science 266, 776-779.; Dawson, P. E., Churchill, M. J., Ghadiri, M. R., and Kent, S. B. H. (1997) Modulation of reactivity in native chemical ligation through the use of thiol additives. J. Am. Chem. Soc. 119, 4325-4329.) Briefly, native chemical ligation reaction is commonly used as a method to obtain a long peptide chain by condensing two peptide segments through the use of a peptide having a thioesterified C terminus and another peptide having Cys introduced at the N erminus. In the first step, the thioester at the C terminus enters an ester exchange reaction with thiophenol to be converted to a more reactive thioester (Figure 5(a)). Then, the thiol group in the N-terminal Cys nucleophilically attacks the carbonyl group of the active ester (Figure 5(b)) and, thereafter, spontaneous S,N acyl rearrangement takes place to form an amide bond at the same site as in the native C34 peptide (Figure 5(c) and (d)). In NCL, the C terminus of a peptide can be used in conjugation and the reaction can be performed under a mild condition (i.e., around pH neutrality), so this method is particularly suitable as a method for synthesizing the peptide of the present invention. It is believed that the C34 peptide derivative of the present invention and the template compound according to the present invention have reacted by the mechanism depicted in Figure 6 to generate the C34 peptide derivative trimer.

The method for carrying out the above-described chemical ligation reaction may advantageously be exemplified by a method in which the template compound according to the present invention and the C34 peptide derivative synthesized according to the present invention are stirred in a buffer, more preferably a 0.1 M sodium phosphate buffer (60 µL, pH 8.5, containing 6 M urea and 2 mM EDTA) having tris(2-carboxyethyl)phosphine hydrochloride (TCEP-HCl) (773 µg, 2.67 µmol) and thiophenol (9 µL, 89 µmol) dissolved therein, whereby the C3-symmetric template compound having three equivalent linker structures and the aforementioned C34 peptide derivative are ligated to synthesize a trimer of the C34 peptide derivative. Such a native chemical ligation reaction occurs in a manner specific for the ligation site (advantageously a thioester group) at the C terminus of the C34 peptide derivative and the Cys in the template compound according to the present invention. The course of the reaction can be monitored using HPLC and ESI-TOF-MS to detect the completion of the reaction.

### 2. Peptide capable of inducing an HIV's three-dimensional structure-recognizing antibody

The peptide the present invention which induces an HIV's three-dimensional structure-recognizing antibody (hereinafter sometimes referred to simply as the "peptide of the present invention") is not particularly limited as long as it is a peptide inducing an HIV's three-dimensional structure-recognizing antibody that recognizes a trimer region of C34, wherein three molecules of a derivative of a helical region C34 peptide at C-terminal region of transmembrane protein gp41 of an HIV particle are ligated via a C3-symmetric template compound having three equivalent linker structures, and an advantageous example is a peptide (trimer of the C34 peptide derivative) represented by the following general formula (4) (where X is a positive integer, and Monomer signifies a compound represented by the following general formula (5)). It is at G that Monomer binds to the non-Monomer portion of the comound of the general formula (4).

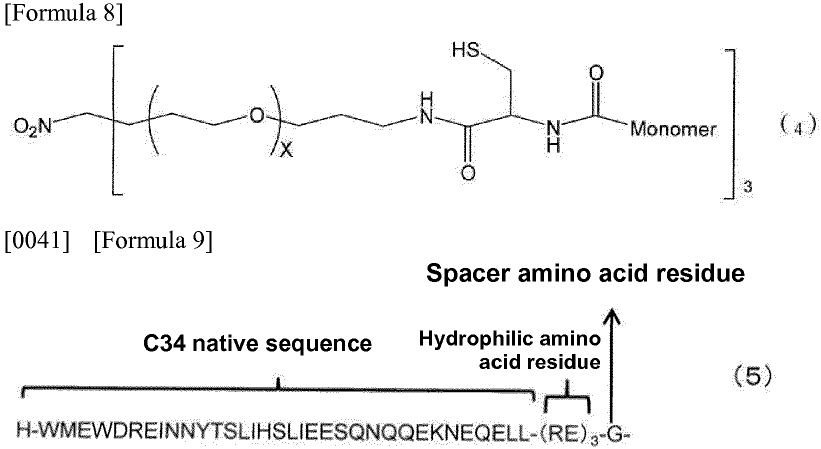

Referring to the above general formula (4), X is preferably an integer within the range of 1 to 10 from the viewpoint of obtaining a trimer of the C34 peptide derivative that is more analogous to the trimeric structure of C34 in the native gp41 protein; X is more preferably an integer within the range of 2 to 8, even more preferably an integer within the range of 2 to 6, still more preferably an integer within the range of 3 to 5, and particularly preferably 4.

### 3. Method for inducing an HIV's three-dimensional structure-recognizing antibody and the HIV's three-dimensional structure-recognizing antibody

The method of the present invention for inducing an HIV's three-dimensional structure-recognizing antibody (hereinafter sometimes referred to simply as the "induction method of the present invention") is not particularly limited as long as it is a method in which a host animal is sensitized with the peptide of the present invention to induce an antibody against said peptide (which is hereinafter sometimes referred to simply as the "antibody of the present invention"). The method of using the peptide to induce an antibody against it may be of any conventionally known type. The antibody according to the present invention which is induced by the induction method of the present invention is an HIV's three-dimensional structure-recognizing antibody that recognizes a trimer region of C34. The antibody of the present invention includes a human antibody. In this context, the "human antibody" according to the present invention means an antibody which is an expression product of a human-derived antibody gene. The human antibody can be obtained by administering the peptide of the present invention to transgenic animals that contain an introduced human antibody gene locus and are capable of producing human-derived antibodies. Examples of the transgenic animals include mice. Examples of mice capable of producing human antibodies include mice that are deficient in endogenous mouse immunoglobulin (Ig) heavy chains and mouse κ-light chains and which possess a human Ig heavy chain gene-containing chromosome 14 fragment (SC20) and a human Ig κ chain transgene (KCo5) at the same time. These mice are prepared by crossing between mice of lineage A having the human Ig heavy chain gene locus and mice of lineage B having the human Ig κ chain transgene. The lineage A is a mouse lineage that is homozygous for both disruptions of endogenous Ig heavy and κ-light chains and which possesses the chromosome 14 fragment (SC20) that can be transmitted to progeny (Tomizuka. et al., Proc Natl Acad Sci USA., 2000, Vol. 97: 722). The lineage B is a mouse lineage that is homozygous for both deficiencies of endogenous mouse Ig heavy and κ-light chains and which possesses the human Ig κ chain transgene (KCo5) (Nat Biotechnol., 1996 Vol. 14: 845).

Moreover, polyclonal or monoclonal antibodies or functional fragments thereof are encompassed in the antibody according to the present invention as long as they are an HIV's three-dimensional structure-recognizing antibody that recognizes a trimer region of C34. The functional fragment of the antibody of the present invention means a fragment of an antibody that specifically binds to an antigen to which the antibody of the present invention binds specifically. More specific examples include F(ab')2, Fab', Fab, Fv, disulphide-linked FV, single-chain FV (scFv), and polymers thereof (D.J. King, Applications and Engineering of Monoclonal Antibodies, 1998, T.J. International Ltd.) Such antibody fragments can be obtained by a routine method, for example, protease (e.g., papain or pepsin) digestion of antibody molecules, or a genetic engineering approach known in the art.

The polyclonal antibody of the present invention can be produced, for example, by the method described below. It is obtained by immunizing non-human mammals such as mice, rabbits, goats, or horses with the peptide of the present invention, optionally together with an immunostimulant (Freund's adjuvant, etc.) The monoclonal antibody of the present invention can be obtained by preparing hybridomas from antibody-producing cells obtained from an immunized animal and myeloma cells having no ability to produce autoantibodies, cloning the hybridomas, and selecting clones that produce monoclonal antibodies that exhibit specific affinity for the antigen used in the immunization. The preparation of said hybridomas can be performed according to the method of Kohler and Milstein, et al. (Nature, 1975, Vol. 256: 495-497) or a method equivalent thereto. The monoclonal antibody-producing hybridoma clones can be screened by culturing the hybridomas in, for example, microtiter plates, and assaying the reactivity with immunizing antigen of a culture supernatant in wells where proliferation has been observed, using an immunological method such as enzyme immunoassay (e.g., ELISA), radioimmunoassay, or a fluorescent antibody method.

To produce monoclonal antibodies from the hybridomas, the latter are cultured *in vitro* and the monoclonal antibodies can be isolated from the culture supernatant. Alternatively, the hybridomas are cultured *in vivo,* for example, in the ascitic fluids of mice, rats, guinea pigs, hamsters, rabbits, or the like, and the monoclonal antibodies can also be isolated from the ascitic fluids. In another method, a monoclonal antibody-encoding gene is cloned from antibody-producing cells such as hybridomas and incorporated in appropriate vectors, with which a host (e.g., mammalian cell lines such as Chinese hamster ovary (CHO) cells, *E. coli,* yeast cells, insect cells, and plant cells) is then transformed. Recombinant antibodies can be prepared from the hosts using a gene recombination technique (P. J. Delves, ANTIBODY PRODUCTION ESSENTIAL TECHNIQUES, 1997 WILEY; P. Shepherd and C. Dean, Monoclonal Antibodies, 2000 OXFORD UNIVERSITY PRESS; J. W. Goding, Monoclonal Antibodies: principles and practice, 1993 ACADEMIC PRESS).

Furthermore, transgenic cow, goats, sheep, or pigs containing the gene of the antibody of interest incorporated in the endogenous gene are prepared using a transgenic animal preparation technique, and antibodies derived from the antibody gene can be obtained at a large scale from the milk of the transgenic animals.

The produced antibodies can be purified by appropriate combinations of methods well known in the art, for example, chromatography using protein A columns, ion-exchange chromatography, hydrophobic chromatography, ammonium sulfate precipitation, gel filtration, and affinity chromatography.

### 4. Preventive and/or therapeutic agent for HIV infectious disease

The preventive and/or therapeutic agent for HIV infectious disease of the present invention (hereinafter referred to simply as the "agent of the present invention") is not particularly limited as long as it comprises the antibody of the present invention or the peptide of the present invention as an active ingredient. The antibody of the present invention as induced by the peptide of the present invention has superior anti-HIV activity or superior neutralizing activity against HIV infection, so the agent of the present invention can be used advantageously as a preventive and/or therapeutic agent for HIV infectious disease. The agent of the present invention encompasses a preventive and/or therapeutic vaccine for HIV infectious disease that comprises the peptide of the present invention as an active ingredient.

To prepare a preventive and/or therapeutic agent for HIV infectious disease using the antibody of the present invention or the peptide of the present invention as an active ingredient to be contained in the agent of the present invention, dosage forms usually adopted with preventive and/or therapeutic agents for infectious diseases comprising a peptide or an antibody as an active ingredient can be adopted. For example, dosage forms or adjuvants or additives usually adopted with preventive and/or therapeutic agents or vaccines for infectious diseases comprising a peptide or an antibody as an active ingredient can be used.

The content of the antibody of the present invention or the peptide of the present invention which are to be incorporated in the agent of the present invention is not particularly limited as long as the desired effect of preventing or treating HIV infectious disease is obtained, and it may be adjusted to lie between 0.00001 and 90 mass%, preferably between 0.0001 and 70 mass%, more preferably between 0.001 and 50 mass%, of the total amount of the agent.

The method of administering the agent of the present invention is not particularly limited as long as the desired effect of preventing or treating HIV infectious disease is obtained, and examples include intravenous administration, intramuscular administration, and subcutaneous administration. In addition, the dosage, frequency and concentration of the administration of the agent of the present invention can be adjusted as appropriate for the body weight and the like of the subject to which it is administered.

The HIV infectious disease as referred to in the present invention is not particularly limited as to whether it has any symptom or how severe it is, and as long as it is a state of infection with HIV, it may be of the acute stage, the asymptomatic stage, or AIDS. The agent of the present invention has the preventive effect against HIV infectious disease and this effect includes both an effect of preventing HIV infection itself and an effect of preventing the onset of AIDS. The agent of the present invention also has the therapeutic effect for HIV infectious disease and this effect includes one of alleviating symptoms derived from HIV infection, particularly one of alleviating symptoms of AIDS in an advantageous embodiment.

The mammalian animals as the subject to which the agent of the present invention is to be administered are not particularly limited but advantageous examples include humans, monkeys, mice, rats, hamsters, guinea pigs, cow, pigs, horses, rabbits, sheep, goats, cats, and dogs, with humans being a more advantageous example.

Other modes of the present invention may be exemplified by the antibody or peptide of the present invention for use in the prevention and/or treatment of HIV infectious disease, a method of using the antibody or peptide of the present invention in the prevention and/or treatment of HIV infectious disease, as well as a method for preventing and/or treating HIV infectious disease by administering the antibody or peptide of the present invention to a subject. The contents of the language in these inventions and preferred modes thereof are the same as have been described on the foregoing pages.

Hereinafter, the present invention will be described more specifically by means of reference examples and working examples. However, the scope of the present invention is not intended to be limited to that of the working examples set forth below.

### EXAMPLE 1

### [Design and synthesis of a C34 peptide derivative trimer]

### (1) Design of a C34 peptide derivative trimer

The C34 peptide derivative trimer as the antigen molecule of interest was of such a design that a peptide mimicking the C34 peptide (C34 peptide derivative) and a template compound on which to provide three of such peptides were synthesized separately and later conjugated (Figure 7). The C34 peptide, in the process of forming a 6-helical bundle with the N36 peptide, is assumed to interact with the N36 peptide starting from the N terminus of the C34 peptide. Therefore, the present inventors presumed that in order to induce an antibody that inhibits HIV membrane fusion at an earlier stage, an antibody recognizing the N-terminal region of the C34 peptide would be more important. Hence, in order to keep the N-terminal region of the C34 peptide in a state more analogous to the native state, the present inventors chose the C terminus of C34 as a site for conjugation with the C3-symmetric template compound (Figure 7).

### (2) Design and synthesis of a C34 peptide derivative

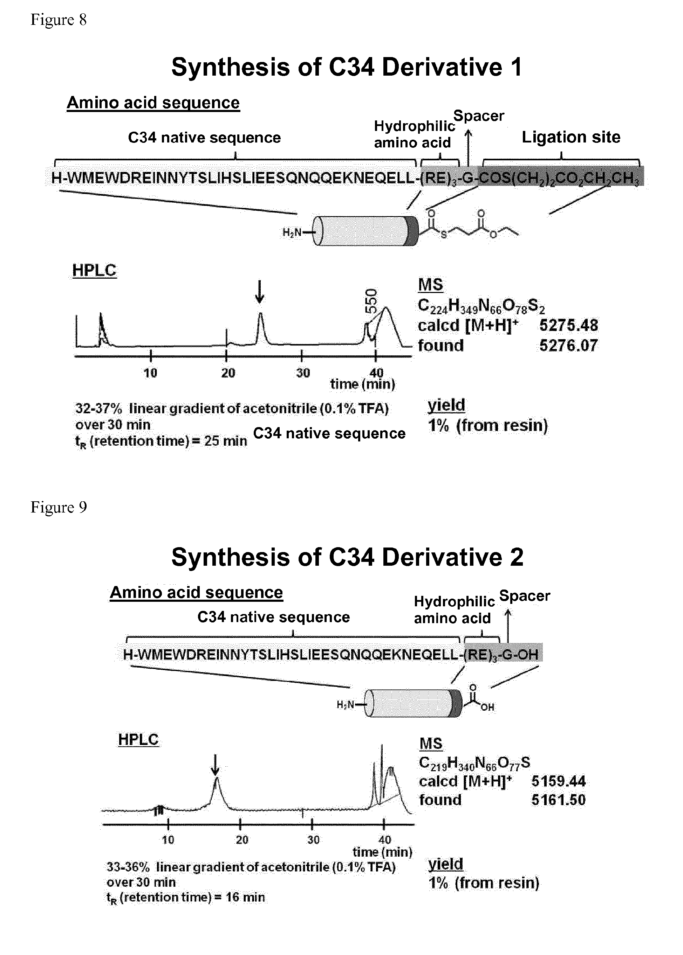

As a C34 peptide derivative for use as a constituent fragment of a C34 peptide derivative trimer, C34 peptide derivative 1 (C34 derivative 1) was designed (Figure 8), and as an antigen molecule for use as a comparison mimicking a C34 peptide monomer, C34 peptide derivative 2 (C34 derivative 2) was designed (Figure 9). The C34 derivative 1 had the following features: to provide improved water solubility, a repeating sequence consisting of the hydrophilic amino acids Arg(R) and Glu(E) were imparted at the C terminus of the C34 native amino acid sequence; in addition, to reduce the steric hindrance that might occur in the process of conjugation with the template compound, Gly(G) was introduced as a spacer at the C terminus of the repeating sequence of hydrophilic amino acids; what is more, the C34 derivative 1 was so designed that the C terminus was thioesterified in order to introduce a ligation site required for conjugation with the C3-symmetric template (Figure 8). On the other hand, the C34 derivative 2 which was not be conjugated with the template compound was different from the C34 derivative 1 in that a ligation site was not introduced in its sequence (Figure 9).

The C34 derivative 1 and the C34 derivative 2 were synthesized and identified by the following specific methods. Chemical reagents for peptide synthesis that contained Fmoc-protected amino acids were purchased from Novabiochem, Inc., KOKUSAN CHEMICAL CO., LTD., and WATANABE CHEMICALL INDUSTRIES, LTD. and used to synthesize C34 related peptides using NovaSyn (registered trademark) TGR resin (0.23 mmol/g, 0.20 mmol scale). Subsequently, solid-phase peptides were synthesized in accordance with the manual. As side-chain protected amino acids, Boc was used for Lys, Pbf for Arg, OBu^{t} for Asp and Glu, Trt for Asn and Gln, and Bu^{t} for Ser, Thr, and Tyr. All peptides were prepared by the Fmoc-chemical method. Each cycle consisted of (i) 15-min deprotection (20% piperidine/DMF) and (ii) 90-min coupling with 5 eq. of Fmoc-amino acid (Fmoc-AA-OH), HOBt and DIPCI in DMF. Coupling efficiency was examined by the Kaiser ninhydrin test and the result was negative, suggesting that the presence of free amino acids was less than 0.05%. When the result of the Kaiser test was weakly positive, the coupling step was repeated (double coupling) using a mixture of Fmoc-amino acid (3 eq.), HOBt (3 eq.), DIPEA (6 eq.) and HBTU (2.9 eq.) After the construction of protected peptides was completed, the resin was fully washed (DMF and MeOH) and vacuum-dried for 6 hours.

### [C34 derivative 2]

To synthesize the C34 derivative 2, the synthesized peptide was cleaved from the resin by treatment with a liquid mixture of TFA, thianisole, ethanediol, m-cresol, H₂O and triisopropylsilane (10/0.75/0.75/0.25/0.25/0.1, v/v) (40 mL) at room temperature for 90 minutes. The resulting liquid reaction mixture was filtered and the resin was washed with TFA (50 mL X 3). The filtrate and the washings were vacuum evaporated to precipitate the peptide, which was washed with cold Et₂O three times. The resulting crude peptide was purified by reverse-phase HPLC to give the C34 derivative 2.

### [C34 derivative 1]

To synthesize the C34 derivative 1, the synthesized peptide was cleaved from the resin by treatment with a liquid mixture of TFE, acetic acid and dichloromethane (1/1/3, v/v) (67 mL) at room temperature for 120 minutes. The resulting liquid reaction mixture was filtered and the resin was washed with dichloromethane three times. The filtrate and the washings were vacuum evaporated to precipitate the peptide as a solid. The unpurified, protected peptide (522 mg) as dissolved in DMF (1.5 mL) was thioesterified with ethyl 3-mercaptopropionic acid (20 eq.), HOBt - H₂O (10 eq.) and 1-(3-dimethylaminopropyl)-3-ethylcarbodiimide (EDCI - HCl) (10 eq.) at 0°C for 1.5 hours. The resulting liquid reaction mixture was stirred overnight at room temperature and concentrated under reduced pressure. To the residue, H₂O (50 mL) was added and the resulting precipitate was washed with H₂O three times. The resulting peptide (692 mg) was treated with TFA/ thianisole/m-cresol/H₂O/triisopropylsilane (TIS) (8.81/0.50/0.32/0.32/0.05, v/v) (30 mL) at room temperature for 2 hours. By vacuum evaporation, the unpurified peptide-thioester was precipitated with cold Et₂O (50 mL), washed with cold Et₂O three times, and purified by reverse-phase HPLC to give the C34 derivative 1.

The purified two peptides, C34 derivative 1 and C34 derivative 2, were freeze-dried and an attempt was made to identify them by ESI-TOF-MS, which was implemented as follows. ¹H NMR spectra were recorded with a Bruker AV500 spectrometer. Chemical shifts were indicated in δ (ppm) relative to the internal standard Me₄Si (in CDCl₃). Using JMS-T1000LC AccuTOF and Brucker Daltonics microTOF-2focus, low- and high-resolution mass spectra were recorded in positive and negative detection modes. Flash chromatography was performed using Wakogel C-200 (product of Wako Pure Chemical Industries, Ltd.) and silica gel 60 N (product of KANTO KAGAKU). In analytical HPLC, Cosmosil 5C18-ARII column (4.6 x 250 mm; product of nacalai tesque) on JASCO PU-2089 plus (product of JASCO Corporation) was run with a linear gradient of 0.1% (v/v) TFA containing CH₃CN at a flow rate of 1 cm³/min⁻¹, and the eluting fractions were detected with uv light at 220 nm. In preparative HPLC, Cosmosil 5C18-ARII column (10 x 250 mm; product of nacalai tesque) on JASCO PU-2089 plus (product JASCO Corporation) was run with an appropriate gradient mode of 0.1% (v/v) TFA containing CH₃CN at a flow rate of 3 cm³/min⁻¹. The results of ESI-TOF-MS as performed on the C34 derivative 1 and the C34 derivative 2 under those conditions are shown below.
C34 derivative 2: m/z calcd for C₂₁₉H₃₄₁N₆₆O₇₇S[M+H]⁺: 5159.5, found: 5160.1 (5.3 mg, yield 1 %).
C34 derivative 1: m/z calcd for C₂₂₄H₃₄₉N₆₆O₇₈S₂[M+H]⁺: 5275.5, found: 5275.6 (11.3 mg, yield 1%).

Thus, each of the C34 derivative 2 and the C34 derivative 1 was obtained at a yield of 1%.

### (3) Design and synthesis of the template compound

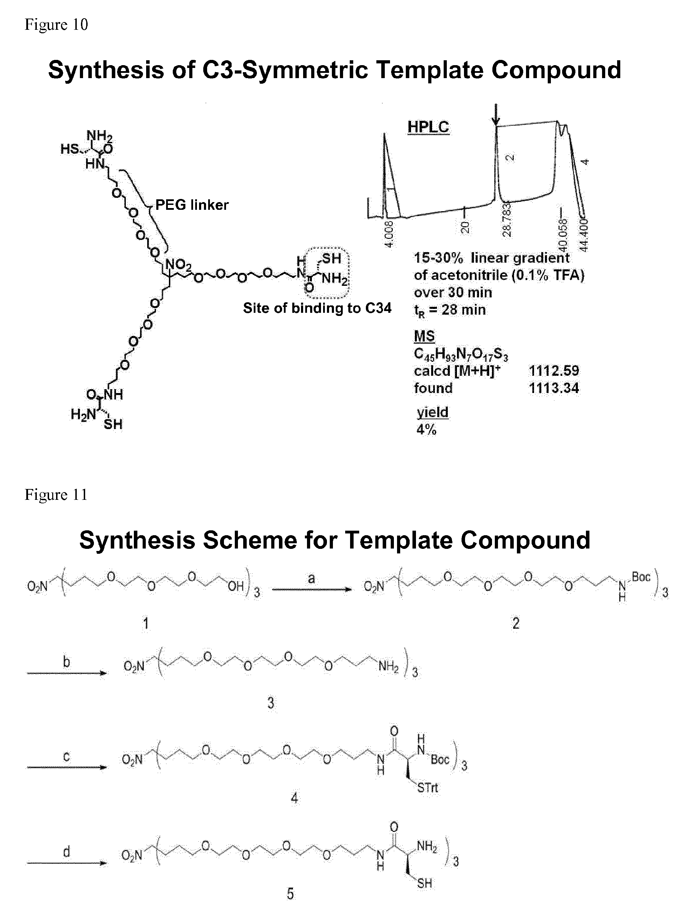

The template compound was so designed that it would be introduced at the C terminus of the C34 peptide. The template compound was designed to have the feature of C3 symmetry to ensure that it would faithfully reflect the structure of the native C34 peptide trimer. As a linker for this template compound, a polyethylene glycol (PEG) group was introduced to provide improved water solubility, and there was introduced Cys necessary for condensation with the C-terminal thioester in the C34 derivative 1 (Figure 10). This template compound was synthesized by the scheme depicted in Figure 11. Specifically, the following method was employed.

### 1) Synthesis of compound 2

Compound 2 in Figure 11 was synthesized as follows. A dry THF solution (4 mL) of tris-[3- {2-(bis-[2-ethoxy])-ethanoyl}-propyloxy]-nitromethane (439 mg, 0.69 mmol) was added to dry THF (2 mL) containing NaH (60% dispersion in mineral oil; 208 mg, 5.21 mmol) at 0°C over 10 minutes. The resulting liquid mixture was stirred at 0°C for 10 minutes. To the liquid reaction mixture, a dry THF solution (1.5 mL) containing (3-bromo-propyl)-carbamic acid tert-butyl ester (992 mg, 4.17 mmol) was added at 0°C over 10 minutes. The resulting liquid reaction mixture was stirred overnight at room temperature and filtered through a Celite pad. The filtrate was concentrated under reduced pressure and chromatographed on silica gel using CHCl₃-MeOH (50/1) to give the titled compound 2 as a colorless oil (425 mg, yield 88%).
¹H-NMR (400 MHz, CDCl₃) δ = 1.44 (s, 27H), 1.48 (m, 6H), 1.76 (q, 6H), 1.95-1.99 (m, 6H), 3.22-3.25 (m, 6H), 3.44 (t, 6H), 3.52-3.66 (m, 42H); ¹³C-NMR (400 MHz) δ = 156.0 (3C), 94.1, 78.7 (3C), 70.6 (3C), 70.5 (3C), 70.5 (3C), 70.1 (3C), 70.1 (3C), 69.5 (3C), 66.5 (3C), 38.5 (3C), 32.2 (3C), 29.6 (3C), 28.4 (9C), 24.0 (3C), 22.2 (3C); HRMS (ESI) m/z calcd for C₅₂H₁₀₂N₄NaO₂₀[M+Na]⁺: 1125.6985, found: 1125.6980.

### 2) Synthesis of compound 3

Compound 3 in Figure 11 was synthesized as follows. To 4 M of HCl/1,4-dioxane (1 mL), compound 2 (118 mg, 0.11 mmol) was added and the resulting solution was stirred at room temperature for 5 hours. By concentrating under reduced pressure, crude compound 3 was obtained as a colorless oil (104 mg) and subjected to the next step without being purified.

### 3) Synthesis of compound 4

Compound 4 in Figure 11 was synthesized as follows. To dry DMF (0.45 mL) were added compound 3 (95.9 mg, 0.12 mmol) and triethylamine (0.17 mL, 1.18 mmol) and the resulting liquid mixture was stirred at room temperature for 10 minutes; to the liquid reaction mixture, a dry DMF solution (0.40 mL) containing Boc-Cys(Trt)-OH (183 mg, 0.39 mmol) and HOBt•H₂O (60.3 mg, 0.39 mmol) was added and stirred at room temperature for 10 minutes. To the liquid mixture, EDCI•HCl (75.5 mg, 0.39 mmol) was added and stirred overnight at room temperature. After diluting the resulting liquid mixture with ethyl acetate, it was washed with saturated aqueous NaHCO₃ and salt water and then dried over Na₂SO₄. After concentrating under reduced pressure, the mass was chromatographed on silica gel using CHCl₃-MeOH (30/1) to give compound 4 as a colorless oil (147 mg, yield 58%). ¹H-NMR (400 MHz, CDCl₃) δ = 1.45-1.49 (m, 6H), 1.57-1.60 (m, 6H), 1.73 (q, 6H), 1.92-1.96 (m, 6H), 3.29 (m, 6H), 3.43 (t, 6H), 3.52-3.62 (m, 42H), 4.98 (s, 3H), 7.19-7.41 (m, 45H); ¹³C-NMR (400 MHz) δ = 170.2 (3C), 144.5 (3C), 129.6 (27C), 128.0 (18C), 126.8 (9C), 94.1, 80.1 (3C), 70.7 (3C), 70.6 (3C), 70.5 (6C), 70.5 (6C), 70.2 (3C), 69.4 (3C), 67.0 (3C), 37.5 (3C), 34.3 (3C), 32.2 (3C), 29.0 (9C), 28.3 (3C), 24.0 (3C); HRMS (ESI) m/z calcd for C₁₁₈H₁₅₉N₇NaO₂₃S₃[M+Na]⁺: 2161.0547, found: 21621.0543

### 4) Synthesis of compound 5

Compound 5 (template compound of interest) in Figure 11 was synthesized as follows. To a liquid mixture of TFA, H₂O, ethanedithiol and triisopropylsilane (90/5/2.5/2.5, v/v), compound 4 (103 mg, 0.048 mmol) was added and the resulting solution was stirred at room temperature for 3 hours and concentrated under reduced pressure. To the residue, Et₂O (15 mL) was added. The resulting precipitate was recovered by centrifugation and the supernatant was removed. Thereafter, the precipitate was washed with Et₂O three times. Purification by preparative HPLC gave compound 5 (template compound of interest) as a colorless oil (28.8 mg, yield 54%).
¹H-NMR (400 MHz, CDCl₃) δ = 1.41 (s, 27H), 1.44-1.48 (m, 6H), 1.76 (q, 6H), 1.95-1.97 (m, 6H), 2.94-3.04 (m, 6H), 3.19-3.36 (m, 6H), 3.48-3.63 (m, 48H), 4.07 (t, 3H); ¹³C-NMR (400 MHz) δ= 167.7 (3C), 70.3, 69.6 (15C), 69.4 (3C), 69.2 (3C), 68.3 (3C), 54.6 (3C), 36.7 (3C), 31.6 (3C), 28.2 (3C), 24.8 (3C), 23.1(3C); HRMS (ESI) m/z calcd for C₄₆H₉₄N₇O₁₇S₃[M+H]⁺: 1112.5868, found: 1112.5858

### (4) Synthesis of C34 peptide derivative trimer

The C34 derivative 1 synthesized in the above-described Example 1(2) and the template compound synthesized in the above-described Example 1(3) were provided and then the C34 derivative 1 was conjugated to the template compound. For conjugation, native chemical ligation (NCL) method was chosen (Dawson, P. E., Muir, T. W., Clark-Lewis, I., and Kent, S. B. H. (1994) Synthesis of proteins by native chemical ligation. Science 266, 776-779.; Dawson, P. E., Churchill, M. J., Ghadiri, M. R., and Kent, S. B. H. (1997) Modulation of reactivity in native chemical ligation through the use of thiol additives. J. Am. Chem. Soc. 119, 4325-4329.) An outline of NCL method is shown in Figure 5. It is believed that the synthesized template compound and C34 derivative 1 reacted by the mechanism depicted in Figure 6 to generate the C34 peptide derivative trimer (C34 Trimer).

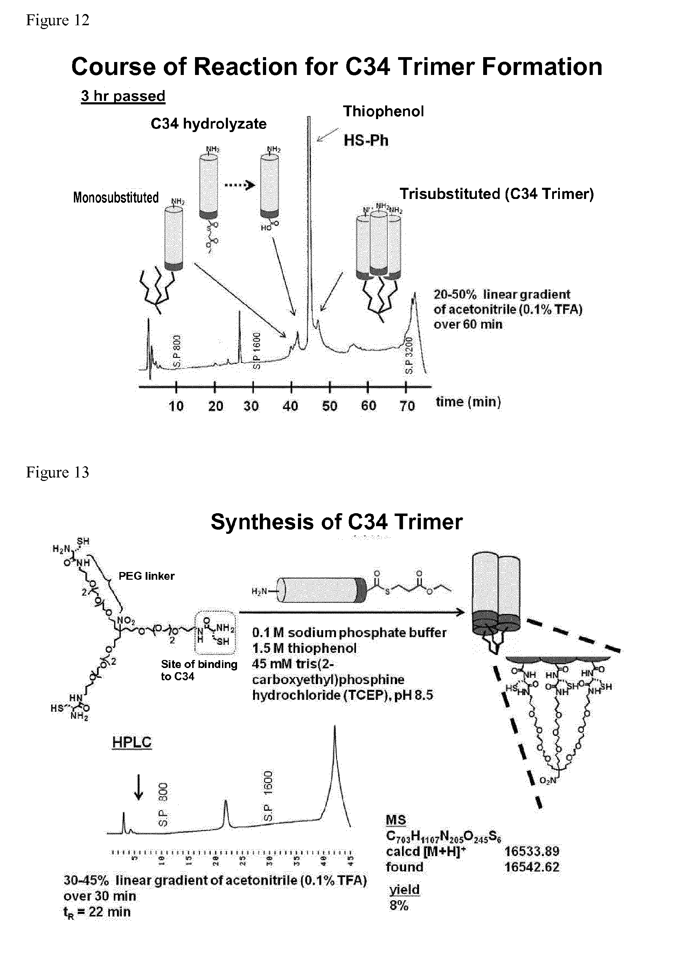

The C34 peptide derivative trimer was synthesized and identified by the following specific methods. TCEP•HCl (773 µg, 2.67 µmol) and thiophenol (9 µL, 89 µmol) were dissolved in 0.1M sodium phosphate buffer (60 µL, pH8.5, containing 6 M urea and 2 mM EDTA) in a nitrogen atmosphere; to the resulting solution, compound 5 as the template compound (100 µg, 0.090 µmol), C34 derivative 1 (1.77 mg, 0.30 µmol) and acetonitrile (20 µL) were added. Reaction was carried out at 37°C for 5 hours under stirring while the course of the reaction was monitored by HPLC. When 3 hours passed after the start of the reaction, an HPLC chart was taken and shown in Figure 12. The C34 peptide derivative trimer was separated as a single peak. Reverse-phase HPLC was performed to purify this C34 peptide derivative trimer. In reverse-phase HPLC, elution using a 33-43% linear gradient of acetonitrile (0.1 % TFA) was performed for 40 minutes. The obtained HPLC chart is included in Figure 13. The purified C34 peptide derivative trimer was obtained in a yield of 17%. This purified C34 peptide derivative trimer was identified by ESI-TOF-MS. m/z calcd for C₇₀₃H₁₁₀₈N₂₀₅O₂₄₅S₆[M+H]⁺: 16533.9, found: 16542.6

To cap the thiol group of the cysteine residue, the C34 peptide derivative trimer (368 µg, 0.02 µmol) was treated with 0.1 M sodium phosphate buffer (74 µL, pH 7.8, containing 6 M urea and 5 mM EDTA) containing iodoacetamide (777 µg, 4.2 µmol) at room temperature. Since no reaction was observed in HPLC and ESI-TOF-MS analyses, it was suggested that the thiol group in the cysteine residue was nonreactive. To determine whether the formation of the dimer or oligomer was due to the disulfide bridge between cysteine residues in the C34 peptide derivative trimer, the molecular weight of the C34 peptide derivative trimer was measured both under a reducing and a non-reducing condition. As a result, it was suggested that under the buffer condition, most of the C34 peptide derivative trimer occurred as a monomer (a single C34 peptide derivative trimer.)

### EXAMPLE 2

### [Secondary structural analysis of the C34 peptide derivative trimer]

To perform a secondary structural analysis of the C34 peptide derivative trimer, a CD (circular dichroism) spectrum was measured. The specific procedure was as follows. The C34 peptide derivative trimer obtained in the above-described Example 1 (final concentration: 2 µM) was dissolved in PBS (50 mM sodium phosphate, 150 mM NaCl, pH 7.2). The resulting solution was set on a J-720 CD spectrometer equipped with thermoregulator (product of JASCO Corporation) and the wavelength dependency of molar ellipticity [θ] was observed at 25°C and 195-250 nm. Another measurement of CD spectrum was performed by the same method, except that the C34 peptide derivative trimer (final concentration: 2 was replaced by the C34 peptide derivative monomer (C34 derivative 2) obtained in the above-described Example 1 (final concentration: 6 µM). The results are shown in Figure 14(A).

The CD spectrum for the C34 peptide derivative trimer (the solid line in Figure 14(A)) and that for the C34 peptide derivative monomer (the dashed line in Figure 14(A)) both showed a minimum value at a wavelength of about 200 nm. Since a negative maximum at about 200 nm characterizes a random coiled structure, it was suggested that the C34 peptide derivative trimer and monomer formed a random coiled structure. It was reported in previous literature that an N36 monomer (N36RE) and an N36 trimer (triN36e) formed a high degree of α-helicity, with triN36e having a higher helix content than N36RE (Nakahara, T., Nomura, W., Ohba, K., Ohya, A., Tanaka, T., Hashimoto, C., Narumi, T., Murakami, T., Yamamoto, N., and Tamamura, H. (2010) Remodeling of dynamic structures of HIV-1 envelope proteins leads to synthetic antigen molecules inducing neutralizing antibodies. Bioconjugate Chem. 21, 709-714.; Chan, D. C., Chutkowski, C. T., and Kim, P. S. (1998) Evidence that a prominent cavity in the coiled coil of HIV type 1 gp41 is an attractive drug target. Proc. Natl. Acad. Sci. U.S.A. 95, 15613-15617.) These results suggested that the C34-derived monomeric and trimeric peptides had a greater tendency to form a random coiled structure than the N36-derived peptides.

To evaluate the interaction between the CD34 peptide derivative trimer and the N36 peptide, the N36 peptide derivative monomer which was an N36 derived peptide (see N36RE depicted below) and the C34 peptide derivative (trimeric or monomeric) were mixed and CD spectra were measured.

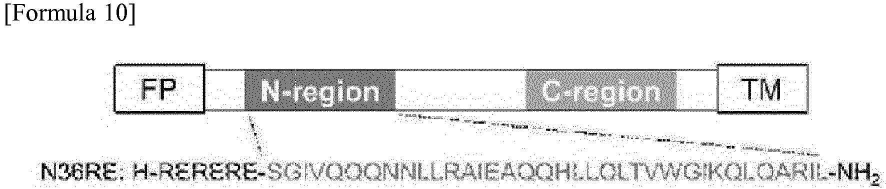

The specific procedure was as follows. A PBS solution containing the C34 peptide derivative monomer (final concentration: 6 µM) and the N36 peptide derivative monomer (final concentration: 6 µM) was provided and a CD spectrum was measured by the same method as described before. For a PBS solution containing the C34 peptide derivative trimer (final concentration: 2 µM) and the N36 peptide derivative monomer (final concentration: 6 µM), a CD spectrum was also measured by the same method. Moreover, for a PBS solution containing only the N36 peptide derivative monomer (final concentration: 6 µM), a CD spectrum was also measured by the same method. The results are shown in Figure 14(B). Both in the CD spectrum for the peptide mixture of the C34 peptide derivative monomer and the N36 peptide derivative monomer (the dashed line in Figure 14(B)) and in the CD spectrum for the peptide mixture of the C34 peptide derivative trimer and the N36 peptide derivative monomer (the solid line in Figure 14(B)), a minimum value appeared twice, one at 208 nm and another at 222 nm, indicating that both peptide mixtures formed α-helicity. The results of Figure 14(B) also showed that the peptide mixture of the C34 peptide derivative trimer and the N36 peptide derivative monomer had a smaller helix content than the peptide mixture of the C34 peptide derivative monomer and the N36 peptide derivative monomer. What was supported by this was that in comparison with the C34 peptide derivative monomer, the C34 peptide derivative trimer was fairly difficult to interact with N36 since it had the three peptide chains associated through covalent bonding.

### EXAMPLE 3

### [Antibody inducing experiment with the C34 peptide derivatives]

To confirm the antibody inducing ability of the C34 peptide derivative trimer, an antibody inducing experiment was performed using the C34 peptide derivative trimer and the C34 peptide derivative monomer. The specific procedure was as follows. Male mice (BALB/c lineage, 6-wk old) were purchased from SANKYO LABO SERVICE CORPORATION, INC. and kept in an animal cage under SPF conditions. Freund's incomplete adjuvant and PBS were commercial products purchased from Wako Pure Chemical Industries, Ltd. DMSO (endotoxin-free) was a commercial product purchased from Sigma-Aldrich. The following experimental protocol was approved by the Ethics Committee of Tokyo Medical and Dental University.

A hundred micrograms of the C34 peptide derivative trimer was dissolved in 50 µL of PBS. To the resulting solution, the Freund's incomplete adjuvant (50 µL) was added and mixed together. The resulting liquid mixture was injected subcutaneously into each mouse under anesthesia at days 0, 7, 14, 21, and 28. Blood was drawn one week before the immunization as well as at days 5, 12, 19, 26 and 33 after the immunization. Each of the thus collected blood samples was centrifuged (1500 rpm) at 4°C for 10 minutes to separate serum. The serum was heated at 56°C for 30 minutes to inactivate the complement. Each serum sample was stored at -80°C until use. Additional serum samples were prepared by the same method, except the C34 peptide derivative trimer was replaced by the same weight of the C34 peptide derivative monomer which was dissolved in 1 µL of DMSO rather than 50 µL of PBS.

### EXAMPLE 4

### [Evaluation of the antibody titers of the sera obtained by immunization with the C34 peptide derivatives]

The ELISA method was adopted as a system for evaluating the antibody titers of the sera obtained in the above-described Example 3. Briefly, the sera at day 33 after the immunization were evaluated for antibody titer and selectivity in terms of the serum titer against the synthesized antigen coating. The specific procedures were as follows.

Reagents were first provided. Tween-20 (polyoxyethylene(20)sorbitan monolaurate) and 30 mass% hydrogen peroxide were purchased from Wako Pure Chemical Industries, Ltd.; 2,2-azino-bis(3-ethylbenzothiazoline-6-sulfonic acid) diammonium salt (ABTS) was purchased from Sigma-Aldrich; an anti-mouse IgG(H+L)(goat)-HRP antibody was purchased from EMD Chemicals (San Diego, California). Then, using 25 µL of PBS containing the synthesized peptide (C34 peptide derivative trimer or C34 peptide derivative monomer) at 10 µg/mL, coating of 96-well microplates was performed overnight at 4°C. The coated microplates were washed with deionized water 10 times; thereafter, using 150 µL of a blocking buffer (0.02 mass% of PBST (0.02 mass% Tween-20 containing PBS) containing 5 mass% of skim milk), blocking was performed at 37°C for an hour. The thus treated microplates were washed with deionized water 10 times. In a separate step, the mouse sera obtained in the above-described Example 3 were diluted with 0.02 mass% PBST containing 1 mass% skim milk and a series of 2-fold dilutions ranging from 200 to 409,600 folds were prepared for each serum sample. Each of these dilutions was added into the wells in the aforementioned microplates in 50-µL portions and incubated at 37°C for 2 hours. Then, the microplates were washed with deionized water 10 times. Subsequently, a solution of HRP bound anti-mouse IgG antibody (secondary antibody) diluted 2000 folds with 0.02 mass% PBST was added into each well in 25-µL portions and incubated for 45 minutes. The microplates were then washed 10 times. Subsequently, an HRP substrate solution prepared by dissolving 10 mg of ABTS in 200 µL of an HRP staining buffer (a liquid mixture of 0.5 M citrate buffer (pH 4.0, 1 mL), H₂O₂ (3 µL), and H₂O (8.8 mL)) was added into each well in 25-µL portions. After incubation for 30 minutes, 0.5 M H₂SO₄ was added in an amount of 25 µL per well to quench the reaction and the absorbance of the solution was measured at 405 nm.

By these procedures, the antibody titers of the obtained sera were measured. The results of the case where the microplates were coated with the C34 peptide derivative monomer are shown in Figure 15(A), whereas the results of the case where the microplates were coated with the C34 peptide derivative trimer are shown in Figure 15(B). The solid squares in the graphs of Figures 15(A) and 15(B) indicate the results of using the sera obtained by induction with the C34 peptide derivative monomer whereas the dots indicate the results of using the sera obtained by induction with the C34 peptide derivative trimer.

The concentration of the 50% bound serum dilution of antibodies induced by the C34 peptide derivative monomer was 1.06 x 10⁻³ with respect to the coating of the C34 peptide derivative monomer and 1.30 x 10⁻³ with respect to the coating of the C34 peptide derivative trimer; the ratio of the latter value of concentration to the former value was about 1.2. In contrast, the concentration of the 50% bound serum dilution of antibodies induced by the C34 peptide derivative trimer was 3.15 x 10⁻⁴ with respect to the coating of the C34 peptide derivative trimer and 7.30 x 10⁻³ with respect to the coating of the C34 peptide derivative monomer; the ratio of the latter value of concentration to the former value was about 23. It is noteworthy that despite non-use of any purified monoclonal antibodies in the evaluation protocol described above, the antibodies produced showed structural selectivity depending on whether the antigen used was monomeric or trimeric. From this result, it was strongly suggested that by synthesizing antigens involving a three-dimensional structure, antibodies having structural specificity could be prepared with high efficiency.

### EXAMPLE 5

### [Evaluation of the neutralizing activity against HIV infection of anti-sera induced by the C34 peptide derivative trimer]

To determine whether the C34 peptide derivative trimer has neutralizing activity (inhibitory activity) against HIV infection, a p24 assay was performed on the sera obtained in the above-described Example 3; p24 is a capsid protein constituting the core of HIV-1 and it is known that HIV multiplication in an HIV-infected organism raises the p24 concentration in the organism's serum. Before performing a p24 assay, HIV-1 virus was first prepared. The specific procedure was as follows.

The HIV-1 molecular clone pNL4-3 (clade B; X4-tropic virus) was provided. Next, 293FT cells were seeded on 10% FBS/DMEM (product of WAKO) in a Petri dish (6 cm^{φ}) and cultured to 60% confluency. The cultured 293FT cells were transfected with 10 µg of pNL4-3 by the calcium phosphate method. Following a culture of 48 hours after the transfection, the supernatant of the culture broth was recovered and passed through a filter (0.45 µm^{φ}) to prepare a virus solution. The thus prepared virus solution was quenched with liquid nitrogen and then stored at -80°C or below until use.

The NL4-3 virus containing 50 ng of p24 was allowed to infect MT-4 cells (5 x 10⁴ cells/200 µL) by spinoculation (2100 g) at 4°C for 20 minutes. After washing away the unbound virions, the MT-4 cells were resuspended in 200 µL of a medium containing 10 µL of each of the mouse sera obtained in the above-described Example 3 and then cultured. During the culture, half of the medium was exchanged every 2 or 3 days. At day 7 from the infection, the amount of p24 in the culture supernatant was measured with a p24 ELISA kit (product of PerkinElmer) (Ohba, K., Ryo, A., Dewan, M. Z., Nishi, M., Naito, T., Qi, X., Inagaki, Y., Nagashima, Y., Tanaka, Y., Okamoto, T., Terashima, K., and Yamamoto, N. (2009) Follicular dendritic cells activate HIV-1 replication in monocytes/macrophages through a juxtacrine mechanism mediated by P-selectin glycoprotein ligand 1. J. Immunol. 183, 524-532.) Three measurements were conducted for each serum sample.

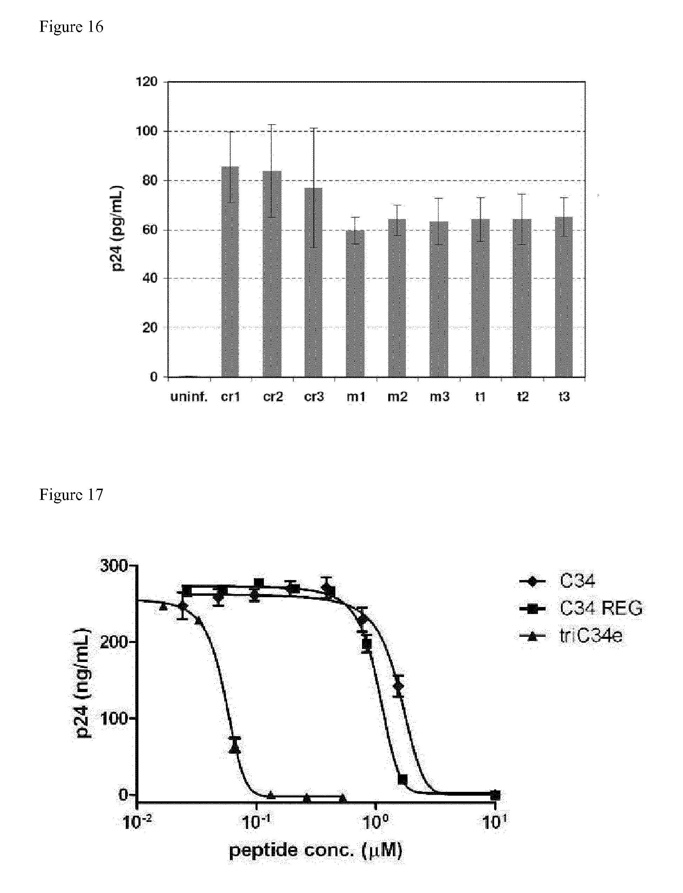

The p24 levels (pg/mL) in the culture supernatant are shown in Figure 16; "uninf." refers to the result from uninfected cells; "cr1" to "cr3" refer to the results from the use of serum one week before immunization (control); "m1" to "m3" refer to the results from the sera induced by C34 peptide derivative monomer; "t1" to "t3" refer to the results from the sera induced by C34 peptide derivative trimer. The sera of the mice immunized the C34 peptide derivative monomer and the sera of the mice immunized with the C34 peptide derivative trimer showed comparable levels of neutralizing activity against viral infection. Since the sera obtained by immunization with the C34 peptide derivative trimer had not only neutralizing activity (see Example 5) but also structural specificity (see the above-described Example 4), said sera, although having a comparable level of neutralizing activity to the sera of the mice immunized with the C34 peptide derivative monomer, are likely to contain a qualitatively different group of antibodies and involve a qualitatively different mechanism of neutralization, so that a structurally specific monoclonal antibody obtained by immunization with the C34 peptide derivative trimer would be more resistant to simple amino acid differences/variations in gp41. Consequently, like the N36 peptide derivative trimer previously discovered by the present inventors (Nakahara, T., Nomura, W., Ohba, K., Ohya, A., Tanaka, T., Hashimoto, C., Narumi, T., Murakami, T., Yamamoto, N., and Tamamura, H. (2010) Remodeling of dynamic structures of HIV-1 envelope proteins leads to synthetic antigen molecules inducing neutralizing antibodies. Bioconjugate Chem. 21, 709-714), the C34 peptide derivative trimer is believed to function effectively as a new class of HIV-1 vaccine.

### EXAMPLE 6

### [Evaluation of the anti-HIV activity of the C34 peptide derivative trimer]

To determine whether the C34 peptide derivative trimer has anti-HIV activity, a virus fusion inhibitory assay was performed using TZM-bl cells. In TZM-bl cells, a luciferase gene linked to the LTR (long terminal repeat) sequence of HIV-1 is incorporated, so infection with HIV-1 can be detected on the basis of the luciferase signal. The virus fusion inhibitory assay was specifically performed by the following method. TZM-bl cells (2 x 10⁴ cells/100 µL) were cultured together with NL4-3 virus containing 5 ng of p24 and a dilution series of peptides. Following 48-hour culture, the TZM-bl cells were lysed and luciferase activity was measured using a Steady-Glo luciferase assay system (product of Promega) (Platt, E. J., Wehrly, K., Kuhmann, S. E., Chesebro, B., and Kabat, D. (1998) Effects of CCR5 and CD4 cell surface concentrations on infection by macrophage tropic isolates of human immunodeficiency virus type 1. J. Virol. 72, 2855-2864.) The EC₅₀ value of each peptide was calculated on the basis of the measured values of luciferase activity. In addition, the CC₅₀ value (µM) of each peptide was calculated on the basis of the decrease in the percent survival of TZM-bl cells in the above-described virus fusion inhibitory assay. The peptides in the dilution series of peptides were the C34 peptide derivative timer (triC34e) and the C34 peptide derivative monomer (C34REG), as well as the C34 native peptide monomer (C34)(C34 peptide derivative monomer minus the repeating sequence of RE and G residue.)

The results for EC₅₀(µM) and CC₅₀(µM) as calculated in the above-described virus fusion inhibitory assay are shown in Table 1. Each of the numerical values given in Table 1 is the mean value of the data obtained by repeating the experiment at least 3 times.

**[Table 1]**

| | C34 | C34REG | triC34e |
|---|---|---|---|
| EC₅₀ (µM)*^{a}* | 0.044 | 0.12 | 0.0013 |
| CC₅₀ (µM)*^{b}* | >15 | >15 | >5 |

As shown by the EC₅₀ data in Table 1, the C34 peptide derivative monomer (C34REG) showed strong enough anti-HIV activity (virus fusion inhibitory activity) but the C34 peptide derivative trimer (triC34e) showed a marked anti-HIV activity which was 100 times more potent. It is noteworthy that the trimeric peptide has a particularly potent anti-HIV activity and this demonstrated the importance of the trimeric morphology as the active structure of the inhibitor. On the other hand, as shown by the CC₅₀ data in Table 1, no cytotoxicity was observed with 15 µM of the C34 peptide derivative monomer (C34REG) and the C34 peptide derivative trimer (triC34e).

### EXAMPLE 7

To determine whether the C34 peptide derivative trimer has anti-HIV activity, an assay was performed using MT-4 cells by the same method as in the above-described Example 6. MT-4 cells (5 x 10,000 cells) were infected with NL4-3 virus containing 1 ng of p24 and incubated at 4°C for 30 minutes in the presence of 5% CO₂. After centrifugation, the culture supernatant was removed and 150 µL of a medium containing serially diluted peptide solutions was added to the cultured MT-4 cells. In the presence of 5% CO₂, incubation was performed at 37°C for 3 days. The p24 concentration (ng/mL) in the culture supernatant after incubation was measured using a commercial ELISA kit (ZeptoMetrix Corp., Buffalo, NY). The results are shown in Figure 17 and Table 2. As can be seen from Figure 17, when the C34 peptide derivative trimer (C34 tri) was used, the p24 concentration dropped markedly as compared with the cases whether the C34 native peptide monomer (C34 peptide) and the C34 peptide derivative monomer (C34 monomer) were used. The EC₅₀ (µM) data calculated from these results are shown in Table 2. Each of the numerical values given in Table 2 is the mean value of the data obtained by repeating the experiment at least 3 times. The C34 native peptide monomer (C34) and the C34 peptide derivative monomer (C34 REG) had EC₅₀ values higher than 1 µM whereas the C34 peptide derivative trimer (triC34e) had an EC₅₀ value of about 50 nM. This showed that the C34 peptide derivative trimer (triC34e) had a superior anti-HIV-1 activity which was about 30 times or about 20 times higher than the activity of the C34 native peptide monomer (C34) or the C34 peptide derivative monomer (C34 REG), respectively.

**[Table2]**

| | C34 | C34 REG | triC34e |
|---|---|---|---|
| EC₅₀ (µM) | 1.59 | 1.06 | 0.0547 |

The C34 peptide derivative monomer fuzeon (T-20/DP178) is a known anti-HIV active peptide that has been put to clinical trial (Non-Patent Document 17). Showing the results obtained by measuring the anti-HIV activities of the C34 native peptide monomer and fuzeon (DP178) by the MAGI assay, Non-Patent Document 17 reports that fuzeon was about 10 times more active than the C34 native peptide monomer in terms of EC₅₀ values. In contrast, as demonstrated by the experimental results of Examples of the subject application, the C34 peptide derivative trimer of the present invention, although assayed by a different method, showed an anti-HIV activity about 30 times higher than that of the C34 native peptide monomer in terms of EC₅₀ values. From these findings, it is presumed that the C34 peptide derivative trimer of the present invention has a superior high anti-HIV activity as compared with fuzeon.

### INDUSTRIAL APPLICABILITY

The present invention is particularly useful in the field of prevention or treatment of HIV infectious disease.

## Claims

1. A method for synthesizing a peptide inducing an HIV's three-dimensional structure-recognizing antibody that recognizes a trimer region of C34, the method comprising:
the steps of synthesizing a derivative of a helical region C34 peptide at C-terminal region of transmembrane protein gp41 of an HIV particle, and
synthesizing a trimer of the C34 peptide derivative by ligating the C34 peptide derivative to a C3-symmetric template compound having three equivalent linker structures.

2. The method for synthesizing a peptide inducing an HIV's three-dimensional structure-recognizing antibody according to Claim 1, wherein the C34 peptide derivative in the step of synthesizing a derivative of a helical region C34 peptide at C-terminal region of transmembrane protein gp41 of an HIV particle is such that a ligation site necessary for ligation with the template compound is introduced at the C-terminal region of a C34 peptide that is the native sequence of C34 whereas spacer amino acid residue(s) for preventing reduced reactivity due to steric hindrance is introduced between the sequence of said C34 peptide and said ligation site.

3. The method for synthesizing a peptide inducing an HIV's three-dimensional structure-recognizing antibody according to Claim 1 or 2, wherein a compound represented by the following general formula (1) or a salt thereof is used as the C3-symmetric template compound having three equivalent linker structures 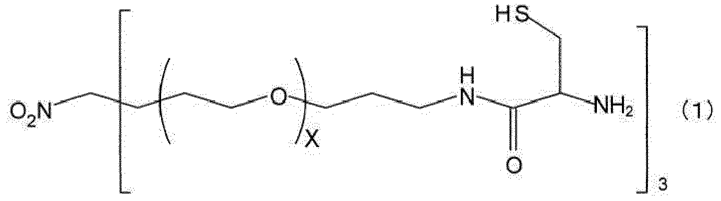 (where X is a positive integer).

4. The method for synthesizing a peptide inducing an HIV's three-dimensional structure-recognizing antibody according to Claim 3, wherein a compound represented by the following general formula (2) or a salt thereof is used as the C3-symmetric template compound having three equivalent linker structures

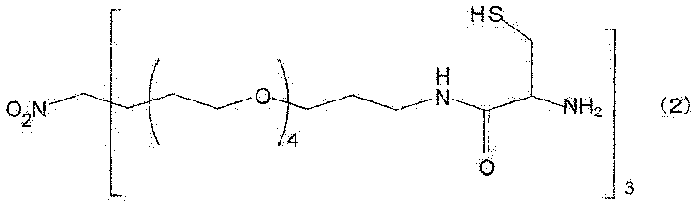

5. The method for synthesizing a peptide inducing an HIV's three-dimensional structure-recognizing antibody according to Claim 1, wherein the C34 peptide derivative in the step of synthesizing a derivative of a helical region C34 peptide at C-terminal region of transmembrane protein gp41 of an HIV particle is such that hydrophilic amino acid residue(s) is imparted at the C terminus of the C34 peptide, a ligation site is further introduced at C terminus thereof whereas spacer amino acid residue(s) for preventing reduced reactivity due to steric hindrance is introduced between said hydrophilic amino acid residue(s) and said ligation site.

6. The method for synthesizing a peptide inducing an HIV's three-dimensional structure-recognizing antibody according to Claim 5, wherein the C34 peptide derivative in the step of synthesizing a derivative of a helical region C34 peptide at C-terminal region of transmembrane protein gp41 of an HIV particle is a compound represented by the following general formula (3)

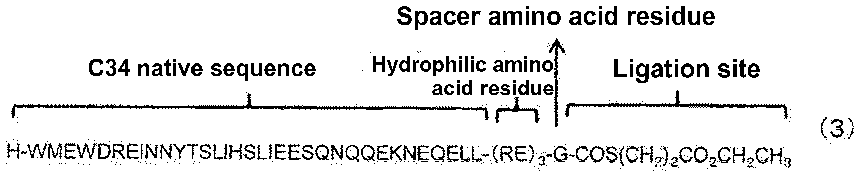

7. The method for synthesizing a peptide inducing an HIV's three-dimensional structure-recognizing antibody according to any one of Claims 1 to 6, wherein the step of synthesizing a trimer of the C34 peptide derivative by ligating the aforementioned C34 peptide derivative to a C3-symmetric template compound having three equivalent linker structures is such that the C3-symmetric template compound having three equivalent linker structures and the C34 peptide derivative are stirred in a buffer, whereby the C3-symmetric template compound having three equivalent linker structures and the aforementioned C34 peptide derivative are ligated to synthesize a trimer of the C34 peptide derivative.

8. A peptide inducing an HIV's three-dimensional structure-recognizing antibody that recognizes a trimer region of a helical region C34 at C-terminal region of transmembrane protein gp41 of an HIV particle, the peptide being synthesized by the method of synthesis according to any one of Claims 1 to 7.

9. A peptide inducing an HIV's three-dimensional structure-recognizing antibody that recognizes a trimer region of C34, wherein three molecules of a derivative of a helical region C34 peptide at C-terminal region of transmembrane protein gp41 of an HIV particle are ligated via a C3-symmetric template compound having three equivalent linker structures.

10. The peptide inducing an HIV's three-dimensional structure-recognizing antibody according to Claim 8 or 9, which is a compound represented by the following general formula (4) or a salt thereof 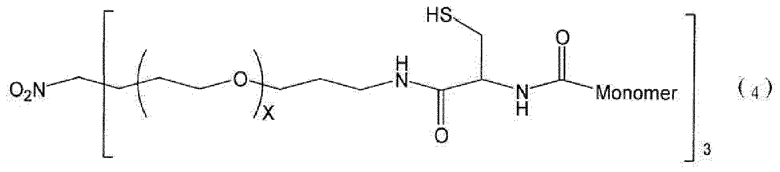 (where X is a positive integer, and Monomer signifies a compound represented by the following general formula (5))

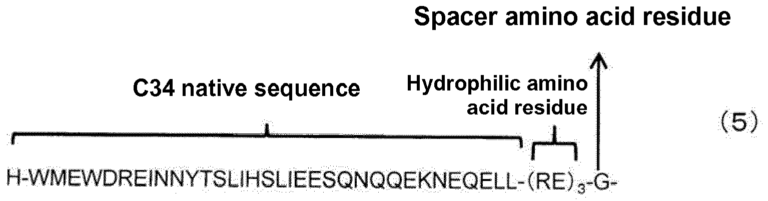

11. A method for inducing an HIV's three-dimensional structure-recognizing antibody that recognizes a trimer region of C34, wherein a host animal is sensitized with a peptide inducing the HIV's three-dimensional structure-recognizing antibody that recognizes a trimer region of C34 according to any one of Claims 8 to 10, whereby an antibody against said peptide is induced.

12. A preventive and/or therapeutic agent for HIV infectious disease comprising as an active ingredient an HIV's three-dimensional structure-recognizing antibody that recognizes a trimer region of C34 as induced by the peptide according to any one of Claims 8 to 10 or the peptide according to any one of Claims 8 to 10.

13. The preventive and/or therapeutic agent for HIV infectious disease according to Claim 12, wherein prevention and/or treatment of HIV infectious disease is due to the anti-HIV activity of the peptide according to any one of Claims 8 to 10.

14. The preventive and/or therapeutic agent for HIV infectious disease according to Claim 12, wherein prevention and/or treatment of HIV infectious disease is due to the action of an HIV's three-dimensional structure-recognizing antibody on a trimer region of a helical region C34 at C-terminal region of transmembrane protein gp41 of an HIV particle, whereby C34/N36 hexamerization of gp41 is inhibited to block the invasion of HIV into a target cell.

15. The preventive and/or therapeutic agent for HIV infectious disease according to Claim 12, which is a preventive and/or therapeutic vaccine for HIV infectious disease comprising as an active ingredient the peptide according to any one of Claims 8 to 10.


SEQUENCE LISTING

<110>  National University Corporation Tokyo Medical and Dental University

<120>  Peptide inducing antibody recognizing conformation of HIV

<130>  P10-040P

<150>  JP 2011-082813
<151>  2011-4-4

<160>  1

<170>  PatentIn version 3.1

<210>  1
<211>  34
<212>  PRT
<213>  Artificial

<220>
<223>  Inventor: Tamamura, Hirokazu ; Narumi, Tetsuo
        Inventor: Nomura, Wataru ; Hashimoto, Chie
        Inventor: Komano, Jun A. ; Miyauchi, Kosuke

<220>
<223>  C34 peptide

<400>  1

Trp Met Glu Trp Asp Arg Glu Ile Asn Asn Tyr Thr Ser Leu Ile His
1               5                   10                  15


Ser Leu Ile Glu Glu Ser Gln Asn Gln Gln Glu Lys Asn Glu Gln Glu
            20                  25                  30


Leu Leu